(19) Europäisches Patentamt / European Patent Office / Office européen des brevets

(11) **EP 3 362 580 B1**

(12) **EUROPEAN PATENT SPECIFICATION**

(45) Date of publication and mention of the grant of the patent:
**17.02.2021   Bulletin 2021/07**

(21) Application number: **16858062.9**

(22) Date of filing: **18.10.2016**

(51) Int Cl.:
*C12Q 1/68* (2018.01)          *G01N 33/483* (2006.01)
*G01N 33/50* (2006.01)          *C12Q 1/6869* (2018.01)
*C12Q 1/6874* (2018.01)          *C12Q 1/6858* (2018.01)
*C12Q 1/6886* (2018.01)          *G16B 20/00* (2019.01)
*G16B 25/00* (2019.01)

(86) International application number:
**PCT/US2016/057496**

(87) International publication number:
**WO 2017/070096 (27.04.2017 Gazette 2017/17)**

(54) **MULTIALLELIC GENOTYPING OF SINGLE NUCLEOTIDE POLYMORPHISMS AND INDELS**

MULTIALLELE GENOTYPISIERUNG VON EINZELNUKLEOTIDPOLYMORPHISMEN UND INDELS

GÉNOTYPAGE MULTIALLÈLES DE POLYMORPHISMES MONONUCLÉOTIDIQUES ET D'INSERTIONS-DÉLÉTIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **18.10.2015   US 201562243078 P**

(43) Date of publication of application:
**22.08.2018   Bulletin 2018/34**

(73) Proprietor: **Affymetrix, Inc.**
**Santa Clara, CA 95051 (US)**

(72) Inventors:
• **GOLLUB, Jeremy Nathan**
**Palo Alto, California 94303 (US)**
• **SHAPERO, Michael H.**
**Campbell, CA 95008 (US)**
• **OLIVER, Dorothy**
**Aptos, California 95003 (US)**
• **BLACK, Deborah**
**Santa Clara, California 95051 (US)**
• **SIDDIQUI, Farooq**
**San Jose, California 95116 (US)**

(74) Representative: **Thermo Fisher Scientific-Life Sciences Solutions Group**
**Life Technologies**
**Frankfurter Straße 129b**
**64293 Darmstadt (DE)**

(56) References cited:
WO-A1-2008/064687          US-A1- 2008 131 894
US-A1- 2012 221 255          US-A1- 2012 221 255
US-A1- 2013 261 196          US-A1- 2014 274 749

• **MERTES ET AL.:** 'Targeted enrichment of genomic DNA regions for next-generation sequencing.' BRIEFINGS IN FUNCTIONAL GENOMICS 26 November 2011, XP055040598

**Description**

FIELD

Aspects described herein generally relate to systems and methods for multiallelic genotyping. In particular, one or more aspects of the disclosure are directed to array-based methods of genotyping multiallelic markers, including single nucleotide polymorphisms (SNPs) and indels, as well as algorithms for determining genotype information of multiple alleles at each variant in samples

BACKGROUND

Synthesized nucleic acid probe arrays, such as Affymetrix.RTM. arrays (Affymetrix, Inc., Santa Clara, Calif.) have been used to generate unprecedented amounts of information about biological systems. For example, arrays can contain probes sufficient to genotype one million single nucleotide polymorphisms (SNPs) per array. Analysis of genotype data from such microarrays may lead to the development of new drugs, new varieties or strains of organisms, including plants, animals, bacteria, archaea and fungi, and new diagnostic tools and treatments based upon genetic information (including information tailored to specific target populations and/or individuals) and the correlation of such information to diseases such as cancer.

US 2012/221255 A1 describes methods of analysis of processed image data from scanned biological probe arrays for the purpose of determining genotype information via identification of Single Nucleotide Polymorphisms.

US 2014/0274749 describes analysis of processed image data from scanned biological probe arrays for determining genotype information via identification of polymorphisms, e.g., Single Nucleotide Polymorphisms (SNPs), insertion/deletion polymorphisms (indels), whole or partial chromosome deletions or duplications.

A majority of SNPs and indels (e.g., insertions or deletions of bases) may be biallelic, in which there may be two alleles in a genetic variation. Thus, conventional genotyping methods may be towards biallelic methods for identifying two alleles; however, some genetic variants may have more than two possible alleles. That is, there is an increased interest in genotyping multiallelic variants, in which multiple alternative alleles exist at a single locus as opposed to haplotypes defined by alleles of multiple, biallelic variants. For example, genomic data, such as that obtained from the 1000 Genomes Project, may contain about 400,000 multiallelic SNPs and indels. Microarray plates, such as Affymetrix.RTM. Axiom.RTM. arrays, may contain a panel of dozens of multiallelic variants that have significant impact on drug metabolism depending on which alternate alleles are present in the panel. Therefore, there is a need for have new approaches for identifying multiallelic variants in genotyping.

SUMMARY

The following presents a simplified summary of various aspects described herein. This summary is not an extensive overview, and is not intended to identify key or critical elements or to delineate the scope of the claims. The following summary merely presents some concepts in a simplified form as an introductory prelude to the more detailed description provided below.

Aspects described herein are directed to systems, methods, and algorithms for multiallelic genotyping and other methods described herein. Genotyping methods typically assume one reference allele and one alternative allele for a marker or a genomic variant. The multiallelic genotyping algorithm disclosed herein is extended from conventional genotyping methods to handle multiallelic markers with more than one variant. That is, the methods disclosed herein may genotype multiallelic SNPs and indels by selecting two alleles to consider at each variant for each sample, in order to reduce the number of alleles considered at a time.

According to specific embodiments, methods for genotyping one or more multiallelic markers using a computer system are disclosed herein. Methods may include acquiring signals for one or more multiallelic markers in one or more samples, for each multiallelic marker, clustering the signals for each pair of alleles in a plurality of allele pairs from the one or more samples, resulting in clusters representing each allele pair, for each homozygous cluster representing a homozygous allele pair, collecting signals for an alternative allele for calculation of a background signal for the alternative allele, resulting in a plurality of background signals each representing a respective allele, assigning initial genotype calls for each sample for each allele pair based upon the signals and the background signals, calculating a multivariate normal distribution for each cluster using the initial genotype calls and prior cluster parameters, for each multivariate normal distribution for each cluster, calculating a logarithmic likelihood of membership for each sample, based on the logarithmic likelihood of membership, calculating, for each sample, a probability of membership in each cluster, and assigning a final genotype call to each sample based on the probability of membership.

According to further embodiments, methods for utilizing whole genome amplification and locus-specific multiplex polymerase chain reaction (mPCR) for preparation of amplicons are also disclosed. These methods may be directed to selectively biasing amplification to improve the quality of genotyping data for the desired marker of interest and to reduce the effect of undesired pseudogenes in the resulting data. Methods may include obtaining genomic DNA (e.g., by extraction), applying whole genome amplification to the genomic DNA, and performing locus-specific mPCR to obtain an increased number of amplicons of desired gene variants. The resulting DNA sample may be fragmented and hybridized to an array which may be utilized for multiallelic genotyping. By creating an intentional imbalance or bias towards amplification of the variants of interest, the downstream bioinformatics analysis may be improved.

According to further embodiments, the present disclosure is involved with methods and/or systems and/or devices that may be used together or independently to evaluate biological assay or testing or experiments and provide or evaluate results. In specific embodiments, the disclosure is involved with an information processing device, such as a computer or laboratory equipment, configured with logic instructions or modules to access data and perform steps as described herein. In further embodiments, the invention is involved with logic instructions and/or data recorded on a tangible media.

These and additional aspects will be appreciated with the benefit of the disclosures discussed in further detail below.

BRIEF DESCRIPTION OF THE DRAWINGS

The patent or application file contains at least one drawing executed in color. Copies of this patent or patent application publication with color drawing(s) will be provided by the Office upon request and payment of the necessary fee. A more complete understanding of aspects described herein and the advantages thereof may be acquired by referring to the following description in consideration of the accompanying drawings, in which like reference numbers indicate like features, and wherein:

Figure 1 illustrates an example of a computer system that may be utilized to execute the software of an embodiment of the invention.

Figure 2 illustrates a system block diagram of the computer system of Figure 1.

Figure 3 illustrates example plots for logarithm transformation of allele intensity to contrast and size.

Figure 4 illustrates example plots of samples assigned to clusters in a biallelic genotyping algorithm.

Figure 5 illustrates a high level flowchart for a multiallelic genotyping method.

Figures 6A, 6B, and 6C illustrate example plots of background signal calculations for each allele pair.

Figures 7A, 7B, and 7C illustrate example plots of initial partitioning of a subset of genotyped samples.

Figure 8 illustrates an example of an N-dimensional, Gaussian mixture model for multiallelic genotyping.

Figure 9 illustrates an example plot of multiallelic call rate versus average cluster concordance including all multiallelic probesets.

Figure 10 illustrates example plots of calls and reference genotypes for several converted probesets.

Figure 11 illustrates an example diagram of a flow of steps for the combination of locus specific amplification of a single gene (e.g., CYP2D6) and whole genome amplification (WGA).

Figure 12 illustrates genotyping plots of results obtained from performing the two approaches illustrated in Figure 11.

Figure 13 illustrates an example diagram of the workflow in the disclosed amplification approach in accordance with one or more aspects of the present disclosure.

Figure 14 illustrates an example table of multiplexing primer sets tested for feasibility in accordance with one or more aspects of the present disclosure.

Figure 15 illustrates an example of genotyping results from oligonucleotide spike-in studies in accordance with one or more aspects of the present disclosure.

Figure 16 illustrates an example table of results from a 15-plex mPCR assay in accordance with one or more aspects of the present disclosure.

DETAILED DESCRIPTION

**GENERAL**

The present disclosure has many preferred embodiments and relies on many patents, applications and other references for details known to those of the art.

As used in this application, the singular form "a," "an," and "the" include plural references unless the context clearly dictates otherwise. For example, the term "an agent" includes a plurality of agents, including mixtures thereof.

An individual is not limited to a human being but may also be other organisms including but not limited to mammals, plants, bacteria, or cells derived from any of the above.

Throughout this disclosure, various aspects of this disclosure may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. All references to the function log default to e as the base (natural log) unless stated otherwise (such as log.sub.10).

The practice of the present disclosure may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques may be had by reference to the example herein below. However, other equivalent conventional procedures may, of course, also be used. Such conventional techniques and descriptions may be found in standard laboratory manuals such as Genome Analysis: A Laboratory Manual Series (Vols. I-IV), Using Antibodies: A Laboratory Manual, Cells: A Laboratory Manual, PCR Primer: A Laboratory Manual, and Molecular Cloning: A Laboratory Manual (all from Cold Spring Harbor Laboratory Press), Stryer, L. (1995) Biochemistry (4th Ed.) Freeman, N.Y., Gait, "Oligonucleotide Synthesis: A Practical Approach" 1984, IRL Press, London, Nelson and Cox (2000), Lehninger, Principles of Biochemistry 3rd Ed., W.H. Freeman Pub., New York, N.Y. and Berg et al. (2002) Biochemistry, 5th Ed., W.H. Freeman Pub., New York, N.Y.,.

The present disclosure may employ solid substrates, including arrays in some preferred embodiments. Methods and techniques applicable to polymer (including protein) array synthesis have been described in U.S. Ser. No. 09/536,841, WO 00/58516, U.S. Pat. Nos. 5,143,854, 5,242,974, 5,252,743, 5,324,633, 5,384,261, 5,405,783, 5,424,186, 5,451,683, 5,482,867, 5,491,074, 5,527,681, 5,550,215, 5,571,639, 5,578,832, 5,593,839, 5,599,695, 5,624,711, 5,631,734, 5,795,716, 5,831,070, 5,837,832, 5,856,101, 5,858,659, 5,936,324, 5,968,740, 5,974,164, 5,981,185, 5,981,956, 6,025,601, 6,033,860, 6,040,193, 6,090,555, 6,136,269, 6,269,846 and 6,428,752, in PCT Applications Nos. PCT/US99/00730  (International Publication Number WO 99/36760) and PCT/US01/04285.

Patents that describe synthesis techniques in specific embodiments include U.S. Pat. Nos. 5,412,087, 6,147,205, 6,262,216, 6,310,189, 5,889,165, and 5,959,098. Nucleic acid arrays are described in many of the above patents, but the same techniques are applied to polypeptide arrays.

Nucleic acid arrays that are useful in the present disclosure include those that are commercially available from Affymetrix (Santa Clara, Calif.) under the brand name GeneChip®. Example arrays are shown on the website at affymetrix.com.

The present disclosure also contemplates many uses for polymers attached to solid substrates. These uses include gene expression monitoring, profiling, library screening, genotyping and diagnostics. Gene expression monitoring, and profiling methods may be shown in U.S. Pat. Nos. 5,800,992, 6,013,449, 6,020,135, 6,033,860, 6,040,138, 6,177,248 and 6,309,822. Genotyping and uses therefore are shown in U.S. Ser. No. 60/319,253, 10/013,598, and U.S. Pat. Nos. 5,856,092, 6,300,063, 5,858,659, 6,284,460, 6,361,947, 6,368,799 and 6,333,179. Other uses are embodied in U.S. Pat. Nos. 5,871,928, 5,902,723, 6,045,996, 5,541,061, and 6,197,506.

The present disclosure also contemplates sample preparation methods in certain preferred embodiments. Prior to or concurrent with genotyping, the genomic sample may be amplified by a variety of mechanisms, some of which may employ PCR. See, e.g., PCR Technology: Principles and Applications for DNA Amplification (Ed. H. A. Erlich, Freeman Press, NY, N.Y., 1992); PCR Protocols: A Guide to Methods and Applications (Eds. Innis, et al., Academic Press, San Diego, Calif., 1990); Mattila et al., Nucleic Acids Res. 19, 4967 (1991); Eckert et al., PCR Methods and Applications 1, 17 (1991); PCR (Eds. McPherson et al., IRL Press, Oxford); and U.S. Pat. Nos. 4,683,202, 4,683,195, 4,800,159 4,965,188, and 5,333,675. The sample may be amplified on the array. See, for example, U.S. Pat. No 6,300,070.

Other suitable amplification methods include the ligase chain reaction (LCR) (for example, Wu and Wallace, Genomics 4, 560 (1989), Landegren et al., Science 241, 1077 (1988) and Barringer et al. Gene 89:117 (1990)), transcription amplification (Kwoh et al., Proc. Natl. Acad. Sci. USA 86, 1173 (1989) and WO88/10315), self-sustained sequence replication (Guatelli et al., Proc. Nat. Acad. Sci. USA, 87, 1874 (1990) and WO90/06995), selective amplification of target polynucleotide sequences (U.S. Pat. No. 6,410,276), consensus sequence primed polymerase chain reaction (CP-PCR) (U.S. Pat. No. 4,437,975), arbitrarily primed polymerase chain reaction (AP-PCR) (U.S. Pat. Nos. 5,413,909, 5,861,245) and nucleic acid based sequence amplification (NASBA). (See, U.S. Pat. Nos. 5,409,818, 5,554,517, and 6,063,603, each of which is incorporated herein by reference). Other amplification methods that may be used include: Qbeta Replicase, described in PCT Patent Application No. PCT/US87/00880, isothermal amplification methods such as SDA, described in Walker et al. 1992, Nucleic Acids Res. 20(7): 1691-6, 1992, and rolling circle amplification, described in U.S. Pat. No. 5,648,245. Other amplification methods that may be used are described in, U.S. Pat. Nos. 5,242,794, 5,494,810, 4,988,617 and in US Pub. No. 20030143599, each of which is incorporated herein by reference. In some embodiments DNA is amplified by multiplex locus-specific PCR. In other embodiments, the DNA is amplified using adaptor-ligation and single primer PCR. Other available methods of amplification, such as balanced PCR (Makrigiorgos, et al. (2002), Nat Biotechnol, Vol. 20, pp. 936-9), may also be used.

Additional methods of sample preparation and techniques for reducing the complexity of a nucleic sample are described in Dong et al., Genome Research 11, 1418 (2001), in U.S. Pat. Nos. 6,361,947, 6,391,592.

Methods for conducting polynucleotide hybridization assays have been well developed in the art. Hybridization assay procedures and conditions will vary depending on the application and are selected in accordance with the general binding methods known including those referred to in: Maniatis et al. Molecular Cloning: A Laboratory Manual (2.sup.nd Ed. Cold Spring Harbor, N.Y., 1989); Berger and Kimmel Methods in Enzymology, Vol. 152, Guide to Molecular Cloning Techniques (Academic Press, Inc., San Diego, Calif., 1987); Young and Davism, P.N.A.S, 80: 1194 (1983). Methods and apparatus for carrying out repeated and controlled hybridization reactions have been described in U.S. Pat. Nos. 5,871,928, 5,874,219, 6,045,996 and 6,386,749, 6,391,623.

The present disclosure also contemplates signal detection of hybridization between ligands in certain preferred embodiments. See U.S. Pat. Nos. 5,143,854, 5,578,832; 5,631,734; 5,834,758; 5,936,324; 5,981,956; 6,025,601; 6,141,096; 6,185,030; 6,201,639; 6,218,803; and 6,225,625, in U.S. Patent application 60/364,731 and in PCT Application PCT/US99/06097 (published as WO99/47964).

Methods and apparatus for signal detection and processing of intensity data are disclosed in, for example, U.S. Pat. Nos. 5,143,854, 5,547,839, 5,578,832, 5,631,734, 5,800,992, 5,834,758; 5,856,092, 5,902,723, 5,936,324, 5,981,956, 6,025,601, 6,090,555, 6,141,096, 6,185,030, 6,201,639; 6,218,803; and 6,225,625, in U.S. Patent application 60/364,731 and in PCT Application PCT/US99/06097 (published as WO99/47964).

The practice of the present disclosure may also employ conventional biology methods, software and systems. Computer software products of the disclosure typically include computer readable medium having computer-executable instructions for performing the logic steps of the method of the disclosure. Suitable computer readable medium include floppy disk, CD-ROM/DVD/DVD-ROM, hard-disk drive, flash memory, ROM/RAM, magnetic tapes and etc. The computer executable instructions may be written in a suitable computer language or combination of several languages. Basic computational biology methods are described in, e.g. Setubal and Meidanis et al., Introduction to Computational Biology Methods (PWS Publishing Company, Boston, 1997); Salzberg, Searles, Kasif, (Ed.), Computational Methods in Molecular Biology, (Elsevier, Amsterdam, 1998); Rashidi and Buehler, Bioinformatics Basics: Application in Biological Science and Medicine (CRC Press, London, 2000) and Ouelette and Bzevanis Bioinformatics: A Practical Guide for Analysis of Gene and Proteins (Wiley & Sons, Inc., 2nd ed., 2001).

The present disclosure may also make use of various computer program products and software for a variety of purposes, such as probe design, management of data, analysis, and instrument operation. See, U.S. Pat. Nos. 5,593,839, 5,795,716, 5,733,729, 5,974,164, 6,066,454, 6,090,555, 6,185,561, 6,188,783, 6,223,127, 6,229,911 and 6,308,170. Computer methods related to genotyping using high density microarray analysis may also be used in the present methods, see, for example, US Patent Pub. Nos. 20050250151, 20050244883, 20050108197, 20050079536 and 20050042654.

Additionally, the present disclosure may have preferred embodiments that include methods for providing genetic information over networks such as the Internet as shown in U.S. patent application Ser. Nos. 10/063,559, 60/349,546, 60/376,003, 60/394,574, 60/403,381.

## DEFINITIONS

Nucleic acids according to the present disclosure may include any polymer or oligomer of pyrimidine and purine bases, preferably cytosine, thymine, and uracil, and adenine and guanine, respectively. (See Albert L. Lehninger, Principles of Biochemistry, at 793-800 (Worth Pub. 1982) which is herein incorporated in its entirety for all purposes). Indeed, the present disclosure contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

An oligonucleotide or polynucleotide is a nucleic acid ranging from at least 2, preferably at least 8, 15 or 20 nucleotides in length, but may be up to 50, 100, 1000, or 5000 nucleotides long or a compound that specifically hybridizes to a polynucleotide. Polynucleotides of the present disclosure include sequences of deoxyribonucleic acid (DNA) or ribonucleic acid (RNA) or mimetics thereof which may be isolated from natural sources, recombinantly produced or artificially synthesized. A further example of a polynucleotide of the present disclosure may be a peptide nucleic acid (PNA). (See U.S. Pat. No. 6,156,501). The disclosure also encompasses situations in which there is a nontraditional base pairing such as Hoogsteen base pairing which has been identified in certain tRNA molecules and postulated to exist in a triple helix. "Polynucleotide" and "oligonucleotide" are used interchangeably in this application.

The term "hybridization" as used herein refers to the process in which two single-stranded polynucleotides bind non-covalently to form a stable double-stranded polynucleotide; triple-stranded hybridization is also theoretically possible. The resulting (usually) double-stranded polynucleotide is a "hybrid." The proportion of the population of polynucleotides that forms stable hybrids is referred to herein as the "degree of hybridization." Hybridizations are usually performed under stringent conditions, for example, at a salt concentration of no more than about 1 M and a temperature of at least 25°C. For example, conditions of $5 \times$ SSPE (750 mM NaCl, 50 mM NaPhosphate, 5 mM EDTA, pH 7.4) and a temperature of 25-30° C. are suitable for allele-specific probe hybridizations or conditions of 100 mM MES, 1 M [Na+], 20 mM EDTA, 0.01% Tween-20 and a temperature of 30-50.degree. C., preferably at about 45-50° C. Hybridizations may be performed in the presence of agents such as herring sperm DNA at about 0.1 mg/ml, acetylated BSA at about 0.5 mg/ml. As other factors may affect the stringency of hybridization, including base composition and length of the complementary strands, presence of organic solvents and extent of base mismatching, the combination of parameters is more important than the absolute measure of any one alone. Hybridization conditions suitable for microarrays are described in the Gene Expression Technical Manual, 2004 and the GENECHIP® Mapping Assay Manual, 2004.

The term "fragment" refers to a portion of a larger DNA polynucleotide or DNA. A polynucleotide, for example, may be broken up, or fragmented into, a plurality of fragments. Various methods of fragmenting nucleic acid are well known in the art. These methods may be, for example, either chemical or physical in nature. Chemical fragmentation may include partial degradation with a DNase; partial depurination with acid; the use of restriction enzymes; intron-encoded endonucleases; DNA-based cleavage methods, such as triplex and hybrid formation methods, that rely on the specific hybridization of a nucleic acid segment to localize a cleavage agent to a specific location in the nucleic acid molecule; or other enzymes or compounds which cleave DNA at known or unknown locations. Physical fragmentation methods may involve subjecting the DNA to a high shear rate. High shear rates may be produced, for example, by moving DNA through a chamber or channel with pits or spikes, or forcing the DNA sample through a restricted size flow passage, e.g., an aperture having a cross sectional dimension in the micron or submicron scale. Other physical methods include sonication and nebulization. Combinations of physical and chemical fragmentation methods may likewise be employed such as fragmentation by heat and ion-mediated hydrolysis. See for example, Sambrook et al., "Molecular Cloning: A Laboratory Manual," 3rd Ed. Cold Spring Harbor Laboratory Press, Cold Spring Harbor, N.Y. (2001) ("Sambrook et al.). These methods may be optimized to digest a nucleic acid into fragments of a selected size range. Useful size ranges may be from 25, 50, 75, 100, 200, 400, 700 or 1000 to 500, 800, 1500, 2000, 4000 or 10,000 base pairs. However, larger size ranges such as 4000, 10,000 or 20,000 to 10,000, 20,000 or 500,000 base pairs may also be useful.

"Genome" designates or denotes the complete, single-copy set of genetic instructions for an organism as coded into the DNA of the organism. A genome may be multi-chromosomal such that the DNA is cellularly distributed among a plurality of individual chromosomes. For example, in human there are 22 pairs of chromosomes plus a gender associated XX or XY pair.

The term "chromosome" refers to the heredity-bearing gene carrier of a living cell which is derived from chromatin and which comprises DNA and protein components (especially histones). The conventional internationally recognized individual human genome chromosome numbering system is employed herein. The size of an individual chromosome may vary from one type to another with a given multi-chromosomal genome and from one genome to another. In the case of the human genome, the entire DNA mass of a given chromosome is usually greater than about 100,000,000 bp. For example, the size of the entire human genome is about $3 \times 10^9$ bp. The largest chromosome, chromosome no. 1, contains about $2.4 \times 10^8$ by while the smallest chromosome, chromosome no. 22, contains about $5.3 \times 10^7$ bp.

A "chromosomal region" is a portion of a chromosome. The actual physical size or extent of any individual chromosomal region may vary greatly. The term "region" is not necessarily definitive of a particular one or more genes because a region need not take into specific account the particular coding segments (exons) of an individual gene.

An "array" comprises a support, preferably solid, with nucleic acid probes attached to the support. Preferred arrays typically comprise a plurality of different nucleic acid probes that are coupled to a surface of a substrate in different, known locations. These arrays, also described as "microarrays" or colloquially "chips" have been generally described in the art, for example, U.S. Pat. Nos. 5,143,854, 5,445,934, 5,744,305, 5,677,195, 5,800,992, 6,040,193, 5,424,186 and Fodor et al., Science, 251:767-777 (1991).

Arrays may generally be produced using a variety of techniques, such as mechanical synthesis methods or light directed synthesis methods that incorporate a combination of photolithographic methods and solid phase synthesis methods. Techniques for the synthesis of these arrays using mechanical synthesis methods are described in, e.g., U.S. Pat. Nos. 5,384,261, and 6,040,193. Although a planar array surface is preferred, the array may be fabricated on a surface of virtually any shape or even a multiplicity of surfaces. Arrays may be nucleic acids on beads, gels, polymeric surfaces, fibers such as optical fibers, glass or any other appropriate substrate. (See U.S. Pat. Nos. 5,770,358, 5,789,162, 5,708,153, 6,040,193 and 5,800,992.)

Preferred arrays are commercially available from Affymetrix under the brand names GeneChip® and Axiom®, and are directed to a variety of purposes, including genotyping and gene expression monitoring for a variety of eukaryotic and prokaryotic species. (See Affymetrix Inc., Santa Clara and their website at affymetrix.com.) Other commercially available arrays include Infinium® arrays (Illumina, Inc., San Diego, California) and SurePrint® arrays (Agilent Technologies, Inc., Santa Clara, California).

An allele refers to one specific form of a genetic sequence (such as a gene) within a cell, an individual or within a population, the specific form differing from other forms of the same gene in the sequence of at least one, and frequently more than one, variant sites within the sequence of the gene. The sequences at these variant sites that differ between different alleles are termed "variances", "polymorphisms", or "mutations". At each autosomal specific chromosomal location or "locus" an individual possesses two alleles, one inherited from one parent and one from the other parent, for example one from the mother and one from the father. An individual is "heterozygous" at a locus if it has two different alleles at that locus. An individual is "homozygous" at a locus if it has two identical alleles at that locus.

Polymorphism refers to the occurrence of two or more genetically determined alternative sequences or alleles in a population. A polymorphic marker or site is the locus at which divergence occurs. Preferred markers have at least two alleles, each occurring at frequency of preferably greater than 1%, and more preferably greater than 10% or 20% of a selected population. A polymorphism may comprise one or more base changes, an insertion, a repeat, or a deletion. A polymorphic locus may be as small as one base pair. Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified allelic form is arbitrarily designated as the reference form and other allelic forms are designated as alternative or variant alleles. The allelic form occurring most frequently in a selected population is sometimes referred to as the wildtype form. Diploid organisms may be homozygous or heterozygous for allelic forms. A diallelic or biallelic polymorphism has two forms. A triallelic polymorphism has three forms. A multiallelic polymorphism has two or more forms. A polymorphism between two nucleic acids may occur naturally, or be caused by exposure to or contact with chemicals, enzymes, or other agents, or exposure to agents that cause damage to nucleic acids, for example, ultraviolet radiation, mutagens or carcinogens. Single nucleotide polymorphisms (SNPs) are positions at which at least two alternative bases occur at appreciable frequency (>1%) in the human population, and are the most common type of human genetic variation. Multiallelic markers may include SNPs or indels with three or more possible alleles.

The term "single nucleotide polymorphism probe" or "SNP probe" as used interchangeably herein, and generally understood in the art, refers to a set of one or more oligonucleotides designed to interrogate a particular single nucleotide polymorphism. Such probes are generally identified according to their position on the array, but can also be identified by, e.g., the use of a tag sequence in a barcode fashion, detectable labels, distinguishable solid supports to which the probes are attached, or a variety of other means known in the art. Within certain assays known in the art, such as the Axiom® Assay (Affymetrix, Inc., Santa Clara, CA) or the Infinium® II Assay (Illumina, Inc., San Diego, CA), after hybridization to the sample, an interrogation base complementary to the next base in the sample sequence is added to the SNP probe (which forms a then at least partially double stranded complex with the sample) and a directly or indirectly detectable signal from the added interrogation base is used to determine the identity of the added interrogation base, from which the identity of the relevant allele is determined. The added interrogation base may be added by a variety of techniques known in the art, such as through ligation or single base extension. As is known in the art, certain array assays utilize SNP probes designed from either a forward or reverse perspective relative to the polymorphism and thus, during probe design, a probe can be complementary to a sequence either to the left or the right of the polymorphism. Non-limiting examples of ligation based interrogation approaches are disclosed within US 2008/0131894.

The term "genotyping" refers to the determination of the genetic information an individual carries at one or more positions in the genome. For example, genotyping may comprise the determination of which allele or alleles an individual carries for a single SNP or the determination of which allele or alleles an individual carries for a plurality of SNPs. For example, a particular nucleotide in a genome may be an A in some individuals and a C in other individuals. Those individuals who have an A at the position have the A allele and those who have a C have the C allele. In a diploid organism the individual will have two copies of the sequence containing the polymorphic position so the individual may have an A allele and a C allele or alternatively two copies of the A allele or two copies of the C allele. Those individuals who have two copies of the C allele are homozygous for the C allele, those individuals who have two copies of the A allele are homozygous for the C allele, and those individuals who have one copy of each allele are heterozygous. The array may be designed to distinguish between each of these three possible outcomes. A polymorphic location may have two or more possible alleles and the array may be designed to distinguish between all possible combinations.

A genotype may refer to the information present at a single polymorphism, for example, a single nucleotide polymorphism or single base indel, or the information present at multiple base positions, such as a complex or multi-base indel. For example, if a SNP is biallelic and may be either an A or a C, then if an individual is homozygous for A at that position, the genotype of the SNP is homozygous A or AA. SNPs may also be multiallelic (as opposed to biallelic) and have three or more possible allelic variants. Genotype may also refer to the information present at a plurality of polymorphic positions.

The term "primer" as used herein refers to a single-stranded oligonucleotide capable of acting as a point of initiation for template-directed DNA synthesis under suitable conditions for example, buffer and temperature, in the presence of four different nucleoside triphosphates and an agent for polymerization, such as, for example, DNA or RNA polymerase or reverse transcriptase. The length of the primer, in any given case, depends on, for example, the intended use of the primer, and generally ranges from 15 to 30 nucleotides. Short primer molecules generally necessitate cooler temperatures to form sufficiently stable hybrid complexes with the template. A primer need not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3' downstream primer that hybridizes with the complement of the 3' end of the sequence to be amplified.

The term "prior" as used as a noun herein refers to an estimate of a parameter plus the uncertainty in the distribution of that parameter that is entered into the calculation before any (current) data is observed. This is standard notation in Bayesian statistics. Such values as estimates for genotype cluster center locations and variances may be used as prior values (such as ones obtained from other data sets or user entered quantities).

The term "probe" as used herein refers to a surface-immobilized molecule that may be recognized by a particular target. See U.S. Pat. No. 6,582,908 for an example of arrays having all possible combinations of probes with 10, 12, and more bases. Examples of probes that may be investigated by this disclosure include, but are not restricted to, agonists and antagonists for cell membrane receptors, toxins and venoms, viral epitopes, hormones (for example, opioid peptides, steroids, etc.), hormone receptors, peptides, enzymes, enzyme substrates, cofactors, drugs, lectins, sugars, oligonucleotides, nucleic acids, oligosaccharides, proteins, and monoclonal antibodies. In some embodiments of the present disclosure, a probe may include a glass-bound oligonucleotide, generally of a length of 30 bases. The length of a probe may be adjusted to compensate for high-GC or low-GC target sequences, wherein GC represents the guanine-cytosine content in target sequences. A variable position of the probe may be at or adjacent to a ligation site at the 3' end of the probe, or toward the center of the probe, or away from the ligation site.

The design and use of allele-specific probes for analyzing polymorphisms is described by e.g., Saiki et al., Nature 324, 163-166 (1986); Dattagupta, EP 235,726, Saiki, and WO 89/11548. Allele-specific probes may be designed that hybridize to a segment of target DNA from one individual but do not hybridize to the corresponding segment from another individual due to the presence of different polymorphic forms in the respective segments from the two individuals. Hybridization conditions should be sufficiently stringent that there is a significant difference in hybridization intensity between alleles, and preferably an essentially binary response, whereby a probe hybridizes to only one of the alleles.

## ILLUSTRATIVE EMBODIMENTS

In the following description of the various embodiments, reference is made to the accompanying drawings identified above and which form a part hereof, and in which is shown by way of illustration various embodiments in which aspects described herein may be practiced. It is to be understood that other embodiments may be utilized and structural and functional modifications may be made without departing from the scope described herein. Various aspects are capable of other embodiments and of being practiced or being carried out in various different ways.

Array based genomic analysis generally targets a very large number of SNPs and other polymorphisms each having at least one probeset, wherein a probeset includes a set of oligonucleotide sequences that are used to determine the presence of a particular SNP. For example, probes may be organized into biallelic pairs or sets and multiallelic probe sets, each of which interrogate a target marker. In some systems, many polymorphisms may have two or more different probesets, with each of the different probesets providing a possible genotyping result for the polymorphisms. In one method, an individual sample is exposed to a genotyping array or other probeset system to determine the presence of different polymorphism alleles in the sample. Because most organisms have multiple copies of every chromosome, there may be different alleles detected for the same sample. Thus, a sample is generally characterized by multiple alleles (e.g., 2 or more) of each polymorphism. Determining the multiple alleles for a polymorphism is generally referred to in the art as genotyping or SNP genotyping.

In one example of a recent genotyping array, Axiom® Genotyping Arrays from Affymetrix, Inc. are able to genotype from a customizable selection of between 1,500 and 2.6 million SNPs per array. An entire array may be tiled (populated) with oligonucleotide probes, which may assay thousands of SNPs and genomic probes. The probes bind to labeled DNA from a target sample. Generally, analysis software is used to quantify the brightness of each fluorescing DNA-probe complex on a gridded image. High intensity spots indicate high affinity between the probe and target DNA sequences and are used to decode the genotypes of individual SNPs. Affymetrix provides other arrays, including human, dog, and other mouse arrays.

SNP or polymorphism genotype calling refers to the process of determining at a polymorphism location what alleles are present. In biallelic polymorphisms, there are generally two different base pairs that may be present at a location, which may be referred to as allele A and allele B. The genotype of an SNP is generally one of (A, A), (B, B), or (A, B). The first two genotypes are generally referred to as homogeneous and the last as heterogeneous. In multiallelic polymorphisms, there may be N different base pairs, wherein N may be any number greater than two. For example, if N = 3, there may be three different base pairs that may be present at a location, including allele A, allele B, and allele C. The genotype of a multiallelic SNP may be one of (A, A), (B, B), (A, B), (A, C), (B, C), or (C, C).

There is a need for improved genotyping algorithms and methods for handling the additional variants in multiallelic markers. As a general introduction to the subject matter described in more detail below, aspects described herein are directed towards systems and methods, comprising one or more software programs, logic modules, and data capture systems, for genotyping multiallelic markers. The multiallelic genotyping methods are directed to assigning genotype calls for markers that have two or more possible variants using Bayesian N-allele Genotyping. The Bayesian N-allele Genotyping (BANG) algorithm was developed to genotype multiallelic markers in diploid genomes, and the algorithm is intended to handle an arbitrary number of alleles (N). The BANG algorithm has been tested on about 150,000 probesets for about 100,000 multiallelic markers obtained from 1000 Genomes Project (phase 3) and assayed in 360 samples (HapMap 270 plus LWK). Using reasonable conversion criteria for call rate and concordance to 1000 Genomes, about 40% of the probesets have exhibited good performance in a first-pass analysis without algorithm parameter tuning or SNP-specific priors.

The BANG algorithm may utilize the design of probe and ligation channel pairs that are each specific to exactly one expected allele. For example, Affymetrix's Axiom® Genotyping Arrays employ a two-color, ligation-based assay with oligonucleotide probes on a microarray substrate. Each location on an array is referred to as a feature and contains many instances of a single probe. In some embodiments, a feature may be 5×5 or 6×6 microns in dimension. Each feature in an array may contain many instances of a unique oligonucleotide sequence complementary to a genomic sequence flanking the SNP site. Solution probes bearing attachment sites for one of two dyes, depending on the SNP site base (e.g., A or T, versus G or C) hybridize to the glass probe/target complex, and are then ligated for specificity. The Axiom® two-color system can distinguish between ligation of an A or T versus or a G or C based on the resulting fluorescence moiety.

A unique combination of a probe and ligation channel may be used to determine the allele present in a target sequence. A specific allele present in a target sequence of a sample may be determined using the ligation channels to distinguish between ligation of A or T nucleotides or ligation of G or C nucleotides by the resulting fluorescence moiety. That is, in some embodiments, an allele present in a target sequence of the sample may be determined by ligating differentially labeled oligonucleotides to the plurality of probes on an array to distinguish between ligation of labeled oligonucleotides with A, T, C, or G nucleotides at the 3' end of the labeled oligonucleotides. In other embodiments, an allele present in a target sequence of the sample may be determined by using single base extension of the plurality of the probes on an array with differentially labeled nucleotides to distinguish between extension with A, T, C, or G nucleotides.

Probesets comprising a collection of probes and expected ligation channels may be intended to assay the various possible alleles of specific markers. Furthermore, the BANG algorithm may be implemented upon acquiring intensity data from a number of samples, resulting in signal values per allele per sample.

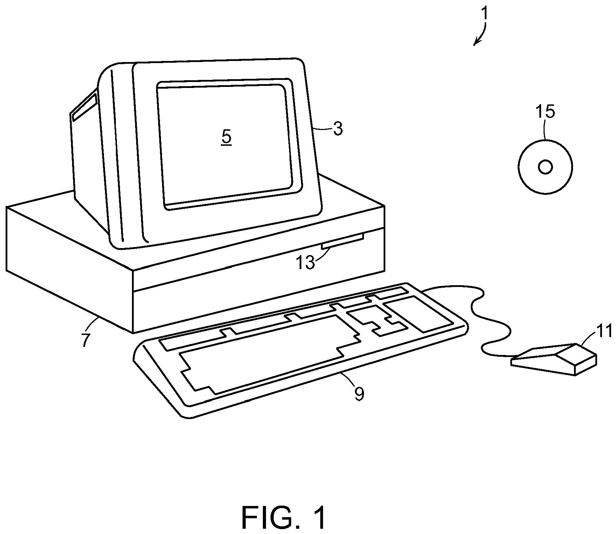

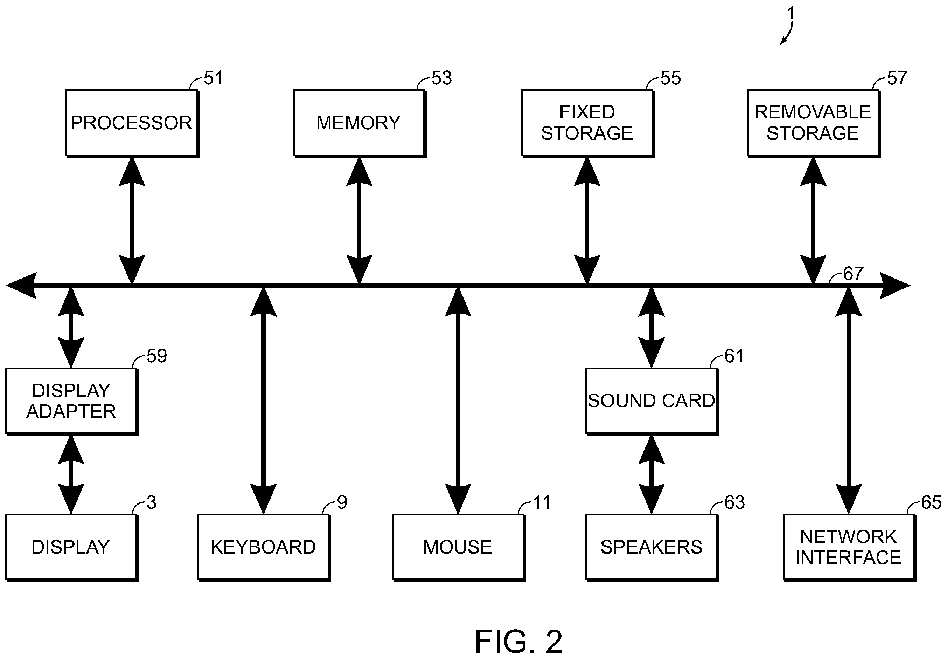

FIG. 1 illustrates an example of a computer system that may be used to execute the software of an embodiment of the invention. FIG. 1 shows a computer system 1 that includes a display 3, screen 5, cabinet 7, keyboard 9, and mouse 11. Mouse 11 may have one or more buttons for interacting with a graphical user interface. Cabinet 7 houses a CD-ROM drive 13, system memory and a hard drive (see FIG. 2) which may be utilized to store and retrieve software programs incorporating computer code that implements the invention, data for use with the invention, and the like. Although a CD-ROM 15 is shown as an exemplary computer readable storage medium, other computer readable storage media including floppy disk, tape, flash memory, system memory, and hard drive may be utilized. Additionally, a data signal embodied in a carrier wave (e.g., in a network including the Internet) may be the computer readable storage medium.

FIG. 2 shows a system block diagram of computer system 1 used to execute the software of an embodiment of the invention. As in FIG. 1, computer system 1 includes monitor 3 and keyboard 9, and mouse 11. Computer system 1 is only one example of a suitable computing system and is not intended to suggest any limitations as to the scope of use or functionality contained in the disclosure. Computer system 1 should not be interpreted as having any dependency or requirement relating to any one or combination of components shown in FIGS. 1 and 2.

Computer system 1 further includes subsystems such as a central processor 51, system memory 53, fixed storage 55 (e.g., hard drive), removable storage 57 (e.g., CD-ROM drive, floppy disk, USB drive), display adapter 59, sound card 61, speakers 63, and network interface 65. Other computer systems suitable for use with the invention may include additional or fewer subsystems. For example, another computer system could include more than one processor 51 (i.e., a multi-processor system) or a cache memory.

The system bus architecture of computer system 1 is represented by arrows 67. However, these arrows are illustrative of any interconnection scheme serving to link the subsystems. For example, a local bus could be utilized to connect the central processor to the system memory and display adapter. Computer system 1 shown in FIG. 2 is but an example of a computer system suitable for use with the invention. Other computer architectures having different configurations of subsystems may also be utilized.

In some aspects, the computer system 1 may include a variety of computer readable media. Computer readable media may be any available media that may be accessed by computer system 1, may be non-transitory, may include volatile and nonvolatile, removable and non-removable media implemented in any method or technology for storage of information such as computer-readable instructions, object code, data structures, program modules, or other data. Examples of computer readable media may include random access memory (RAM), read only memory (ROM), electronically erasable programmable read only memory (EEPROM), flash memory or other memory technology, compact disk read-only memory (CD-ROM), digital versatile disks (DVD) or other optical disk storage, magnetic cassettes, magnetic tape, magnetic disk storage or other magnetic storage devices, or any other medium that may be used to store the desired information and that may be accessed by computer system 1.

Although not required, various aspects described herein may be embodied as a method, a data processing system, or as computer-readable medium storing computer-executable instructions. For example, a computer-readable medium storing instructions to cause a processor to perform steps of a method in accordance with aspects of the disclosed embodiments is contemplated. For example, aspects of the method steps and algorithms disclosed herein may be executed on a processor on computer system 1. Such a processor may execute computer-executable instructions stored on a computer-readable medium.

Software may be stored within memory 53 and/or storage (e.g., fixed storage 55 or removable storage 57) to provide instructions to processor 57 for enabling computer system 1 to perform various functions. For example, memory 53 may store software used by computer system 1, including but not limited to an operating system, application programs, and associated databases. Also, some or all of the computer executable instructions for computer system 1 may be embodied in hardware or firmware. Although not shown, memory 53 may include one or more applications representing the application data stored in memory while the computer system 1 is on and corresponding software applications (e.g., software tasks), are running on computer system 1.

The network interface 65 may allow the computer system 1 to communicate with other devices over any network connections, including local area network (LAN), wide area network (WAN), or other networks. For example, the computer system 1 may establish communications over the Internet or other types of computer networks. In some embodiments, the computer system 1 may communicate with other devices, such as optical scanners which may be used to scan arrays. For example, scanners may image the targets by detecting fluorescent or other emissions from labels associated with target molecules, or by detecting transmitted, reflected, or scattered radiation. A scanner may provide a signal representing intensities (and possibly other characteristics, such as color that may be associated with a detected wavelength) of the detected emissions or reflected wavelengths of light, as well as the locations on the array substrate where the emissions or reflected wavelengths were detected. Typically, the signal includes intensity information corresponding to areas of the scanned substrate. In some embodiments, the computer system 1 may obtain or collect the signals from the scanner (e.g., signal data for all samples and all possible alleles) through the network interface 65 and process the data accordingly to stored instructions.

The disclosure is operational with numerous other general purpose or special purpose computing system environments or configurations. Examples of well-known computing systems, environments, and/or configurations that may be suitable for use with the disclosed embodiments include, but are not limited to, personal computers (PCs), server computers, hand-held or laptop devices, smart phones, multiprocessor systems, microprocessor-based systems, optical scanners, measurement devices/instruments, set top boxes, programmable consumer electronics, network PCs, minicomputers, mainframe computers, distributed computing environments that include any of the above systems or devices, and the like. Computer systems suitable for use with the invention may also be embedded in a measurement instrument.

In some examples, the Bayesian N-allele Genotyping (BANG) algorithm and other genotyping algorithms may be stored and/or implemented on the computer system 1. The multiallelic genotyping algorithm may be applied to acquired intensity data from samples for multiallelic markers.

## ALGORITHM DETAILS

The BANG algorithm may proceed with the following steps. First, the algorithm may estimate background signals for each allele in a plurality of samples. The algorithm may then determine pairs of alleles and genotype appropriate samples using a biallelic genotyping algorithm, such as the Axiom® GT1 or BRLMM-P algorithms from Affymetrix, Inc. or the GenCall software with the GenTrain algorithm from Illumina, Inc., to obtain initial calls for most or all of the samples. Next, conjugate priors may be combined with the signals of corresponding samples to obtain the posterior, multivariate normal distributions of signals corresponding to each diploid genotype cluster, and final assignments of genotypes may be assigned to samples based on likelihood of membership in each distribution.

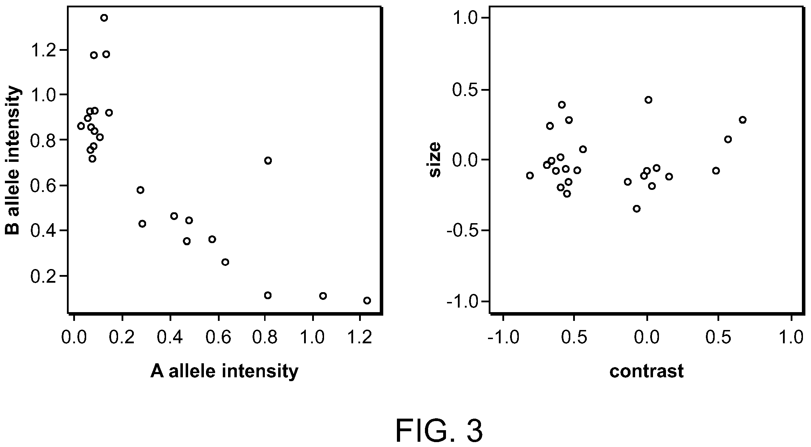

In some embodiments, multiallelic genotyping may leverage biallelic genotyping techniques for identifying allele pairs. For example, in biallelic genotyping, the allele intensity data may be transformed in logarithmic signal space to contrast and size values (e.g., signal strength). Figure 3 illustrates example plots of logarithm transformation of allele intensity to contrast and size. The data used herein is artificial and is simply used for illustration. The following equations may be used to calculate the contrast and size values based on the allele A and allele B intensities.

$$X\ (contrast) = log_2 A - log_2 B$$

$$Y\ (size) = \frac{1}{2}(log_2 A + log_2 B)$$

The transformed intensity data may then be clustered to partition the data for assigning initial calls. For each pair of alleles, signals for those alleles from all samples are clustered using the Axiom® GT1 algorithm, and SNP specific priors and algorithm parameters may be employed. That is, there may be a cluster representing each allele pair.

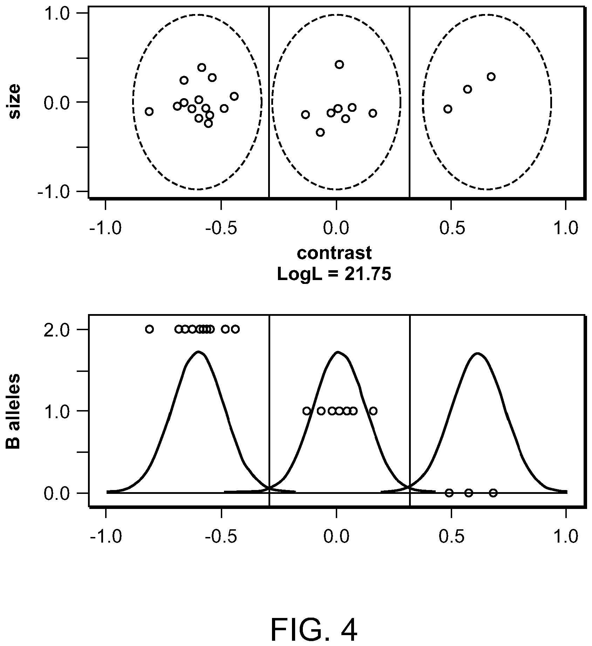

Figure 4 illustrates example plots of samples assigned to clusters in a biallelic genotyping algorithm. The plots in Figure 4 represent clusters for allele pairs BB, AB, and AA, as well as density plotted on the lower graph in two-dimensional space. Based on the plots of the transformed intensities, samples may be assigned to different clusters, and the logarithmic likelihood of the data is computed given the distributions and cluster assignments. For example, the algorithm may evaluate all possible placements of vertical boundaries between data on the X axis and compute for each division a posterior likelihood given the combination of data and a Bayesian prior on cluster locations. Cluster centers and variances may be inferred from the weighted combination of data and priors using the most likely division of data. Additionally, posterior probabilities for each sample in each of the clusters may be computed. Samples that do not match in any of the clusters may be identified and added to an "ocean" cluster, and probabilities may be renormalized. Calls may be assigned to clusters with the highest posterior probability and no call may be assigned if the highest probability is too low.

Multiallelic genotyping may similarly utilize the Axiom® GT1 algorithm to set up initial clusters and assign final genotype calls and confidence levels. However, the multiallelic genotyping algorithm may also extend likelihood calculations to an n-dimensional space and calculate the posterior probability of each sample belonging to each cluster, along with an "ocean" cluster added for samples that do not fit any of the clusters well.

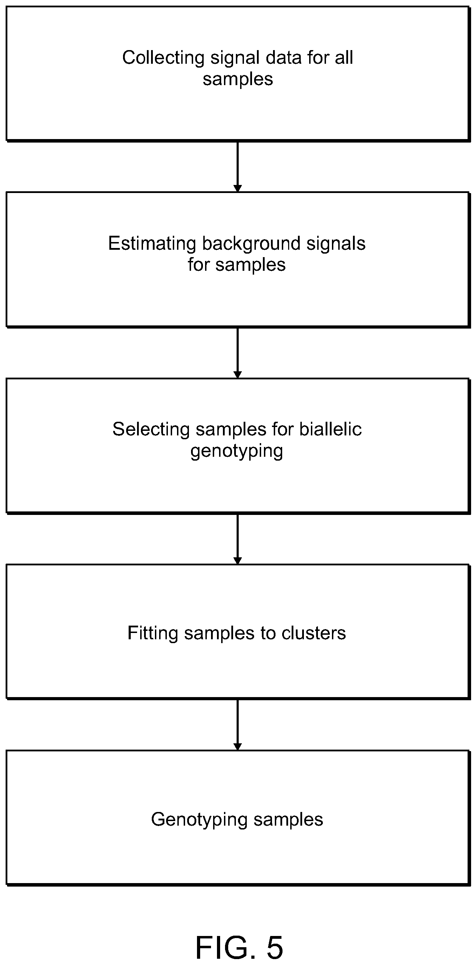

Figure 5 illustrates a high level flowchart for a multiallelic genotyping method. The flowchart in Figure 5 provides an overview of the steps involved in the BANG algorithm.

## SIGNAL COLLECTION AND BACKGROUND ESTIMATION

Signal data may initially be collected for all samples and all possible alleles, in which each sample may have more than two signal values, one per allele. In some embodiments, acquiring the signals for multiallelic markers in the samples may be based on hybridization of the samples with a plurality of probes on an array for measuring the multiallelic markers. The samples may be genotyped using the Axiom® GT1 algorithm in all possible biallelic combinations.

The algorithm may gather metrics for each allele (variant) in a multiallelic marker, skipping markers with less than 3 variants. The alleles in each set may be sorted, and calls and metrics may be gathered for each of the biallelic pairs. Each of the variants being interrogated may be paired into biallelic sets, and each biallelic set may be genotyped. For example, if three alleles A, B, and C are potentially present, all samples may be genotyped three times considering the A/B, A/C, and B/C allele combinations.

For each pair of alleles, the signals for those alleles from all samples may be clustered using the Axiom® GT1 algorithm, and SNP specific priors and algorithm parameters may also be employed.

For each sample assigned to a homozygote cluster, the other-allele signal may be included in a calculation of average background signal for that other allele. For example, in an allele A vs. allele B clustering, the B signals of samples in the AA cluster may be added to the collection of B background signals. Likewise, the A signals of samples in the BB cluster may be added to the collection of A background signals. This process may be repeated for each pair of alleles, and the mean and standard deviation of background signal may be calculated for each allele. In some embodiments, each of the allele background signals may be averaged across all allele pairings, whereas in other embodiments, independent background signal estimates for each allele may be obtained.

If all samples are found to have AA, AB, or AC genotypes, there might not be an estimate of an allele A background signal. In such cases where an allele might not have any background signals, the weighted mean of average background signals of the other alleles, and the weighted mean of their standard deviations, may be used instead. In some embodiments, a global estimated background signal may be used for an allele if no values are available to calculate the mean, variance, and standard deviation of the background signal for the allele. The global estimated background signal may be an average of the plurality of background signals for all the alleles.

Note that in each pairwise clustering, background signals may be selected only for the two alleles in question. That is, in the A vs. B clustering, C background signals might not be selected. A sample may also contribute more than once to the same background estimate, if the sample is called a homozygote of more than one other allele in the various pairwise clusterings.

Specific prior values may be allowed as an option during the background calculation step as these priors may differ from the prior values used during the genotyping round. If priors are not provided, generic values may be used. The signals and the background of the probeset may be calculated if the sample size is greater than 0. Otherwise these metrics may be set to -1 with the exception of the standard deviation for the channel background which may be set to 0 if the sample size is less than 1.

The metrics for each allele of the biallelic pairs may be be derived from the signals of the homozygote calls for the biallelic probeset. The average signal for an allele($avgSig_{allele}$) may be derived by summing the signal of homozygote calls for that allele ($allele_{hom}$) and then dividing by the total number of samples that contributed to these signals ($nsig_{allele}$). The background value of an allele ($bgnd_{allele}$) may be calculated by adding the signal for that allele during homozygote calls when the call does not match the allele ($\sum allele_{in\ hom\ call\ not=allele}$). The average for the background of an allele ($avgBgnd_{allele}$) may be calculated by taking the sum of these signals from the allele divided by the number of signals. Variance ($varianceBgnd_{allele}$) and standard deviation ($stdevBgnd_{allele}$) may also be calculated for the background signal for an allele. The average signal found in the background for a given allele may be added to a multiple of the standard deviation in order to set an individual background threshold for the given allele.

The average signal, background signal, mean, variance, standard deviation of the background signal, and other parameters for each respective allele may be calculated using the following equations:

$$avgSig_{allele} = \sum allele_{hom} / nsig_{allele}$$

*where allele in (A,B,C,D,E,F)*

$$bgnd_{allele} = \sum allele_{in\ hom\ call\ not=allele}$$

$$avgBgnd_{allele} = bgnd_{allele} / nsig_{allele}$$

$$weightedAvgBgnd = \sum (avgBgnd_{allele} * nsig_{allele}) / \sum nsig_{allele}$$

$$varianceBgnd_{allele} = \sum (bgnd_{allele_i} - avgBgnd_{allele})^2 / nsig_{allele} - 1$$

$$stdevBgnd_{allele} = \sqrt{varianceBgnd_{allele}}$$

$$weightedAvgStDevBgnd = \sum(stdevBgnd_{allele} * nsig_{allele})/\sum nsig_{allele}$$

$$bgnd_{allele} = bgnd_{allele} + \text{SIG\_THRESHOLD\_VAR\_MULTIPLE} * stdevBgnd_{allele}$$

The total number of signals that contributed to all the channels may also be calculated. The overall average background (allAvgBgnd) and standard deviation (allAvgStDev) for all the probesets ($n_{ps}$) may be calculated by averaging the weighted average background and weighted average standard deviation values across all probesets in the multiallelic set. An overall weighted average background (allWeightedAvgBgnd) value and overall weighted average standard deviation (allWeightedAvgStDev) may also be calculated by summing up the average values of these metrics and weighing them by the number of samples contributing to the total weight, and then dividing this value by the number of samples. A background threshold for alleles that did not have an individual allele threshold set due to not having signals in the background may be calculated. This calculation may necessitate multiplying the weighted average standard deviation by a set factor specified by the parameter SIG_THRESHOLD_VAR_MULTIPLE (e.g., a current default may be 2). This value may be added to the overall weighted average background.

$$nSignals = \sum nSig_{alleles}$$

$$allAvgBgnd = \frac{\sum_{i=0}^{n_{ps}} weightedAvgBgnd_i}{n_{ps}}$$

$$allStDev = \frac{\sum_{i=0}^{n_{ps}} weightedAvgStDevBgnd_i}{n_{ps}}$$

$$allWeightedAvgBgnd = \sum_{i=0}^{n_{ps}}(weightedAvgBgnd_i * nSignals_i) / \sum_{i=0}^{n_{ps}} nSignals$$

$$allWeightedAvgStDev = \sum_{i=0}^{n_{ps}}(weightedAvgStDev_i * nSignals_i) / \sum_{i=0}^{n_{ps}} nSignals$$

$$BgndThreshold$$
$$= allWeightAvgBgnd + (\text{SIG\_THRESHOLD\_VAR\_MULTIPLE}$$
$$* allWeightedAvgStDev)$$

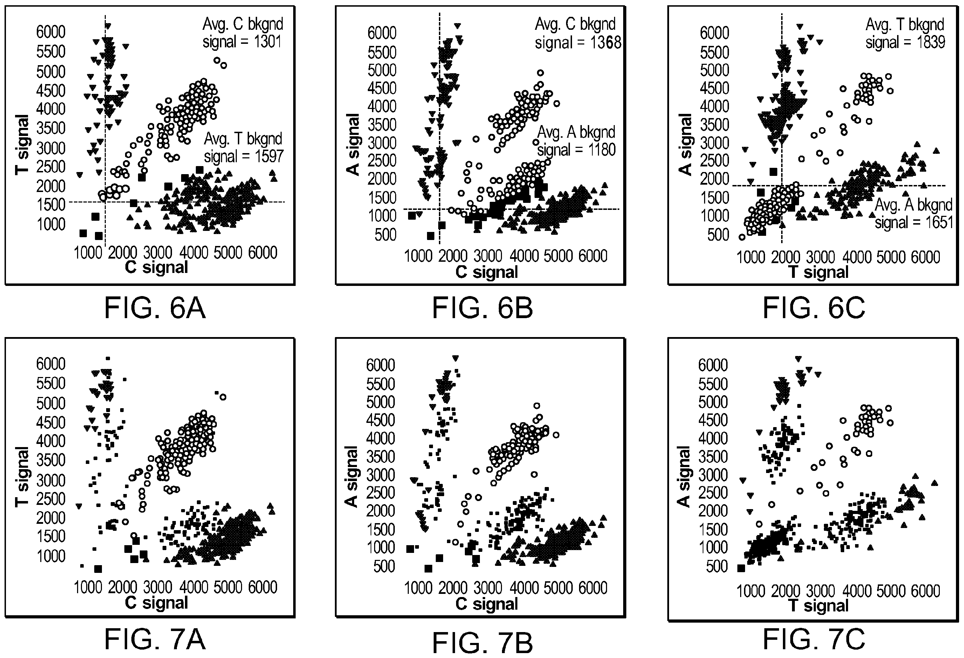

Figures 6A, 6B, and 6C illustrate example plots of background signal calculations for each allele. For each multiallelic marker, all samples may be clustered in all possible biallelic combinations, and the resulting homozygous calls may be used to estimate the mean and variance of the background signals. In the example shown in Figures 6A-6C, rs3091244 is an A/C/T triallelic marker, and the possible biallelic combinations include C allele vs. T allele (Figure 6A), C allele vs. A allele (Figure 6B), and T allele vs. A allele (Figure 6C). The three background signal estimates range from approximately 1,350 to approximately 1,700.

Figures 7A, 7B, and 7C illustrate example plots of initial partitioning of a subset of genotyped samples. For example, a subset of samples may be genotyped in each possible biallelic combination. The resulting calls may be consolidated into tentative multiallelic genotype calls. In Figure 7A, samples with a "high A signal" may be removed from the C allele vs. T allele clustering plot. A "high A signal" may indicate a signal above the allele A background mean plus two standard deviations. In Figure 7B, samples with a high T signal may be removed from a C allele vs. A allele clustering plot, and in Figure 7C, samples with a high C signal may be removed from a T allele vs. A allele clustering plot.

## ALGORITHM SETTINGS

Information for mapping variants to a given marker may be contained in files (e.g., CDF file) accessed during implementation of the algorithm. A program running the algorithm (e.g., a program executing on computer system 1) may read the priors files for the multiallelic markers, as well as settings. Settings for the multiallelic genotyping algorithm may include parameters used during biallclic genotyping, as well as parameters with different initial default values assigned for multiallelic genotyping. An initial call assignment for multiallelic genotyping may have the same parameters and setting available as the biallelic genotyping algorithm. Table 1 below includes parameters that the final call assignment for multiallelic genotyping may have which may differ from the initial step.

Table 1. Parameters for Final Call Assignment for Multiallelic Genotyping

| Parameter | Purpose |
|---|---|
| ocean | Value for uniform density; Test datapoints against uniform ocean probability |
| freqFlag | Apply mixture frequency penalty to clusters (mix) |
| wobble | Cap number of mean observations; limits prior pseudo-observations to 1/wobble |
| lambda | Controls mixing of common variances |
| inflatePRA | Make calls adding uncertainty in mean to observed variance |
| confidence Threshold | Threshold for no calls |
| hardshell | Can stop clusters from being too close |
| shellbarrier | How close clusters can be |

## INITIAL GENOTYPE ASSIGNMENT

After calculating the background signals for each allele, the algorithm may assign initial genotype calls for each sample for allele pair based on the allele signals and the background signals. For example, various biallelic probeset combinations may be genotyped using an object from a class in a program file for the algorithm.

Each allele may have an estimate of a background signal, and a sample may be considered to have a signal above background for an allele if that allele's signal is greater than a predefined threshold value. In some embodiments, the predefined threshold value may be calculated to be equal to $avgBgnd_{allele} + 2*stdevBgnd_{allele}$. For each pair of alleles, the algorithm may identify a subset of samples that do not have a signal above the background signal in any other allele or in any alternative allele. For example, when considering allele A vs. allele B, all samples that do not have signal above background in allele C, allele D, and the like, may be included during genotyping and classified. In other words, during each round of genotyping of biallelic combinations, if one of the other alleles not being genotyped during the round has a signal above a background threshold, then the sample may be excluded from the current genotyping round.

For each pair of alleles, the algorithm may determine if the number of samples in the subset of samples (e.g., samples with no other allele signal above the background) is above a predefined minimum value. For example, if more than a minimum number (e.g., 3 or any number) of appropriate samples have been found, then those samples may be genotyped for the two alleles represented in the corresponding allele pair using the Axiom@ GT1 algorithm, in which specific priors and algorithm parameters may be employed. This determination step may be repeated for each pair of alleles with more than the minimum number of samples found. At the end of the process, samples may have 0, 1, or more calls from the various iterations.

Prior values that might be specific to the biallelic combination currently being genotyped may be allowed as an option though generic values may also be used if specific priors are not provided. The calls, classification statistics, number of biallelic comparisons and indices of the samples genotyped may be stored for all rounds of genotyping (e.g., stored by computer system 1).

## COMBINATION OF BIALLELIC CALLS INTO MULTIALLELIC CALLS

All of the biallelic calls for each sample may be collected, and the collection of calls for each sample may then

14

be resolved to a single, tentative genotype call. For each biallelic call, the two alleles that the probeset is interrogating may be mapped lexicographically to the corresponding multiallelic signals, e.g., A, B, C, D, E, F, etc. For example, if one is interrogating a triallelic A/C/T marker, the A allele would be mapped to the A signal, the C allele to the B signal and the T allele to the C signal. To make a call for the biallelic C/T probeset, the algorithm may map the corresponding biallelic call to a multiallelic call. If the call is a -1, the algorithm may return a "no call value." If the call is a 0, then the returning multiallelic call would be BB which corresponds to having two C alleles. If the call is a 2, then the returning multiallelic call would be CC which corresponds to having two T alleles. If the call is a 1, then BC may be returned which corresponds to a heterozygote CT call. All biallelic calls may be collected for each sample, and the biallelic call that was most often made may be assigned the sample. For example, the algorithm may compare calls for each sample in order to choose a call occurring most frequently in each sample. If there is a tie for the call made most often, the sample may be assigned an inconsistent call. In some embodiments, if there is a tie among the calls, a "no-call" value may be assigned to the sample. If a sample has never been included in any of the iterations, the sample may be assigned a "no-call" value.

## MULTIVARIATE NORMAL DISTRIBUTION AND FINAL GENOTYPE ASSIGNMENTS

After the initial calls have been assigned, the signals may be fit to multivariate normal distributions describing each cluster to determine the likelihood that a sample was derived from a given cluster. That is, the algorithm may write out the summary signals by the alleles and the initial calls, and the files of the summary signals along with a priors file may be read by the program running the algorithm. The signals may be converted into logarithmic signal space, and each probeset may be assigned all possible clusters with the corresponding priors for each of the clusters. The number of observations, mean, and covariance of a cluster may be derived from the initial calls.

In other words, the algorithm may use initial calls and priors (e.g., generic or SNP specific priors trained on other data) to calculate a multivariate normal distribution for each diploid genotype cluster in logarithmic signal space. For each diploid genotype cluster, the priors may be updated with the log2 signals of each sample tentatively assigned to that cluster.

The mean and covariance for the multivariate normal describing a given cluster may be determined using the data for that cluster combined with the prior parameters for that cluster. The conjugate prior used for the multivariate normal distribution may be in the normal-inverse-Wishart form. These prior parameters may be available as input through a file loaded for use with the algorithm. Default values for these parameters may be set through the program in the event that a file is not provided. These parameters may be combined with the cluster data in the following manner (as shown by the equations below):

$$\kappa_1 = \kappa_0 + \eta$$

$$\upsilon_1 = \upsilon_0 + \eta$$

$$\mu_1 = \frac{\kappa_0 \mu_0 + \eta \bar{x}}{k_0 + \eta}$$

$$\Delta_1 = \Delta_0 + S + \frac{\kappa_0 \eta}{\kappa_0 + \eta} (\bar{x} - \mu_0)(\bar{x} - \mu_0)^T$$

$$S = \sum_{i=1}^{n} (x_i - \bar{x})(x_i - \bar{x})^T$$

$\kappa_0$ is the prior number of observations for mean

$\eta$ is the number of observation of data

$\upsilon_0$ is the prior number of observations for variance

$\mu_0$ is the prior mean

$\overline{x}$ *is the sample mean*

$\Delta_0$ *is* $\upsilon_0 \Sigma_0$

In some embodiments, the posterior parameters may be adjusted accordingly. For example, the signal strength in the data may be used to position empty clusters to the expected locations since these positions may differ from the locations specified in the prior file. The average background allele signal and the average allele signal may be gathered from the homozygote signals in the data. For empty clusters, the signal strength may be drawn from the average allele signal then adjusted downward. If the cluster does not contain the allele, the signal may be taken from the background signal for the allele. If the actual allele signal or background signal for the allele is not available, the missing value may be calculated by adding or subtracting the expected amount of difference from the signal that is present. The expected amount of difference between the clusters may be contained in two variables: one to specify the expected distance between cluster with 0 alleles and cluster with 1 allele (copyNumber0to1) and the other to specify the expected distance between a cluster with 1 allele and the cluster with 2 alleles (copyNumber1to2).

Additionally, a final check may be performed to make sure that the clusters are in the right order. For example, a cluster containing the B allele (BB, AB) may have a higher signal for allele B than a cluster that does not contain the B allele (CC, CD). A cluster that contains two alleles may also be expected to have a slightly higher signal for that allele than a cluster that contains only one copy of that allele. For example, BB may have a higher signal in B than cluster AB. The method may also ensure that the distance specified by the two shell barrier values separates the clusters. The shell barrier values may specify the distance between the two clusters in question. The variable "shellbarrier0to1" may be the minimum distance between the clusters with 0 alleles and 1 allele, while "shellbarrier1to2" may be the minimum distance between the cluster with 1 allele and 2 alleles. For the clusters AA, AB and BB and allele A, "shellbarrier0to1" may specify the minimum distance between the A allele's position in the BB cluster and the A allele's position in the AB cluster, while "shellbarrier1to2" may specify the minimum distance between the A allele in the AB cluster and the A allele in the AA cluster.

Thus, if necessary, posterior mean positions may be adjusted to preserve order. For example, the AA cluster may have a greater log2 A signal than the AB, AC, etc. clusters, which may in turn have greater log2 A signal than all clusters that do not include the A allele. Clusters with allele copy number 1 may be compared to clusters with allele copy number 0, and their mean log2 allele signals may be increased (if necessary) to the maximum of copy number 0 log2 allele signals plus S (which may be a configurable parameter). This adjustment may then be repeated comparing homozygote clusters (copy number 2) to heterozygote clusters (copy number 1).

With posterior distributions established, the log likelihood of membership (L) in each distribution (cluster), for each sample, may be calculated as follows:

$$\ln(L) = -\ln(|\Sigma|) - \frac{1}{2}(x - \mu)^T \Sigma^{-1}(x - \mu) - \frac{k}{2}\ln(2\pi)$$

$$\Sigma = \frac{\Delta_1}{\upsilon_1}$$

$\mu = \mu_1$

$|\Sigma|$ *is the determinant of the covariance*

*x is the $\kappa$ - dimensional column vector containing the signal for a sample for a probeset*

*k is the number of signals for the probeset*

The exponentiation of the log likelihood may be calculated followed by rescaling likelihood values either by subtracting the minimum (if working with the negative log likelihood) or subtracting the likelihood from the maximum. An "ocean" adjustment value may be calculated by multiplying the value for the "ocean" parameter to the minimum likelihood value.

The probability of membership in each cluster, or in a uniform "ocean" cluster, may be calculated for each sample from the log likelihoods, and the sample may be assigned to the cluster with the greatest probability. That is, a final genotype call may be assigned to each sample based on the probability of membership for a particular cluster. A confidence value, defined as the probability of membership of the sample in any of the other clusters, may be calculated for each sample and compared to a predefined threshold value. Samples falling above that threshold value may each be assigned a "no-call" value.

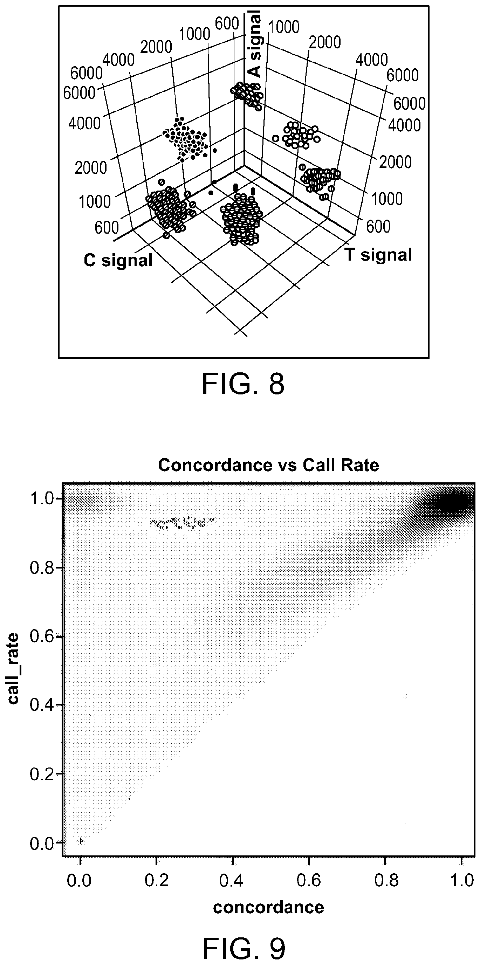

By way of further example, Figure 8 illustrates an example of an N-dimensional, Gaussian mixture model for multiallelic genotyping. The model in Figure 8 may be utilized for assigning final multiallelic calls, and the model may be constructed by combining conjugate priors (generic or trained on real data) with data from the samples tentatively assigned to each genotype. Posterior probability of membership in each genotype cluster may be calculated for each sample, and the genotype with the greatest likelihood may be assigned as the final genotype call if it exceeds a specified threshold. This step may resolve any conflicting genotype calls from the initial partitioning and may produce a meaningful probability of each possible genotype for each sample.

## SOFTWARE IMPLEMENTATION

According to further specific embodiments, one or more of the above methods may be included in a software package to automatically genotype multiallelic markers from array data or similar genotype data. Such a software package may read many different files during execution of the BANG algorithm. Examples of program files include (but are not limited to) the following:

AxiomDMETMultiallelicCaller.java

AxiomDMETClusterer.java

AxiomDMETStem.java

assign_final_calls.py

**AxiomGT1.summary.txt** - the summary file containing the signals of all the biallelic probesets that are in the multiallelic sets

**Probeset file (annotation file)** - the file containing the information as to which multiallelic set a probeset belongs to and whether the probeset is part of a wobble set. The program may skip over probesets in a wobble set as these necessitate consolidation by the AxiomDMETSummarizer.java program before multiallelic calls may be run.

**Reference file** - file containing reference calls. Used for testing purposes.

**Output file name** - this is the name of the file that will contain the calls for a multiallelic probeset. The name is used as a prefix when generating other output files which detail statistics (_ProbeSetSummary.txt) and which may be used with the SpotFire graphing program (_spotfire.txt).

**AxiomGT1.multiallelic_summary.txt** - Summary file generated from the program AxiomDMETMultiAllelicCaller.java

**AxiomGT1.multiallelic_calls.txt** - calls file from the program AxiomDMETMultiAllelicCaller.java

The probeset group (ps_group) may be used to identify the group of probesets that are interrogating the same multiallelic marker. Ideally, there may be a multi_asid assigned to all the probesets in a multiallelic set. There may also be another identifier to identify a wobble set for each set of probesets in a multiallelic set which may need to be consolidated before being run through the multiallelic calling program. The allclcs_fwd may still be needed in order to identify the alleles that a biallelic probeset is interrogating. It may also be helpful to have all the alleles that are present on an array listed for the multiallelic marker (the multi_alleles field). At this point, the program may assign the channel based on the alleles_fwd field. The ideal situation may be to have some mapping between the allele or biallelic probeset_id and the channel in order to separate the data from the implementation.

These may be the necessary data in the annotation file that should be available in the library files:

**probeset_id -** Necessitates a way in which one can identify the different biallelic probesets in a multiallelic set. The current implementation uses the probeset_id for the biallelic probesets in a multiallelic set.

**multiallelic** - Necessitates a way to identify which probesets are multiallelic and thus need to be called using a multiallelic calling algorithm. Current implementation uses a boolean to denote whether the probeset is part of a multiallelic set of probesets (0/1)

**multi_asid -** A multiallelic probeset identifier.

**ps_group -** A means by which the probesets in the multiallelic set can be identified. Currently the probeset group is used for this purpose but any identifier can be used as long as a mapping can be made between the identifier and the probesets in the set as well as a way to identify which pro

**multi_alleles** - all the alleles for a multiallelic marker

**affy_snp_id** - the id for the marker being interrogated by a biallelic probeset

**wobble** - Probes can be used to interrogate markers for all variants close to the target variant (wobbles). These wobble sets needs to be consolidated into a probeset before being genotyped. Thus a means of identifying whether consolidation needs to occur may be necessary. Currently a boolean is used to indicate if a probe is part of wobble set (0/1). The multiallelic calling algorithm currently skips all data for which this boolean is set to 1 as this identifies wobble sets which necessitate consolidation. This will not be needed beyond the prototype since the program will first perform consolidation followed by genotyping.

**alleles_fwd** - - A means by which one can identify each of the biallelic probesets consolidations in a multiallelic set. The alleles being interrogated in the forward direction has to date served this purpose.

**DMETcall** - Stores the calls allowed for a DMET code. Necessitates a way to map the biallelic codes for a call to the corresponding numeric DMET code.

A Summary file may contain the following data: $a\_ij$, the Channel A signal for probeset i and sample j, from summary file; $b\_ij$, the Channel B signal for probeset i and sample j, from summary file.
A reference file may contain the reference call for the multiallelic probeset.

## ADDITIONAL PARAMETERS AND SETTINGS

The following parameters may be considered as possible user settings.
**AxiomDMETMultiAllelicCaller.java** has the following parameters:
**OUTPUT_CALLS_NUMERIC_CODE** - a boolean to designate whether numeric DMET code should be used for calls file
**AxiomDMETStem.java** has the following parameters:

**MIN_LOG2_SIG** - a minimum values for the log 2 signal (currently set to 0.000001)

**SIG_THRESHOLD_VAR_MULTIPLE** - multiple applied to overall weighted average variance used in background threshold calculation.

The **AxiomDMETClusterer.java** program has the following parameters:

**WORKING_DIR_PATH** - path for the temporary files created in order to call and characterize biallelic probesets
**SUMMARY_FILE_NAME** - the name of the summary file that will be created containing the signals from two channels for one biallelic probeset
**POSTERIOR_FILE_NAME** - model file for the biallelic probeset generated by the apt-summary-genotype program for the summary file
**CALLS_FILE_NAME** - the name of the calls file for the biallelic probeset generated from the summary file by apt-summary-genotype f
**METRICS_FILE_NAME** - the name of the metrics file for the biallelic probeset generated by Ps_metrics based on the files generated by apt-summary-gcnotypc
**PERFORMANCE_FILE_NAME** - name of the performance (classification) file for the biallelic probeset created using the metrics file and the other files generated by apt-summary-genotype
**APT_SUMMARY_GENOTYPE** - the actual path for the program
**OUTPUT_DIR_NAME** - name where the genotype results generated by apt-summary-genotype for the biallelic probeset will be stored
**GENOTYPES_FILE_PATH** - path and name of calls file
**PS_CLASS_FILE_PATH** - path and name of performance file
**SCRIPT_NAME** - name of the script used to call apt-summary-genotype, Ps_metrics and Ps_classification on the biallelic probeset data
**CMD** - string for script that will be run on a biallelic probeset if the script does not yet exist

**Probeset Selection:**

Multiallelic calls may be generated for probesets that are designated in the probeset file as being multiallelic and not belonging to a wobble set. A "1" may be used to designate that the probeset is multiallelic and a "0" may be used to designate that the probeset is not part of a wobble set.

**Output files**

In some embodiments, four initial output files may be generated from the multiallelic genotyping algorithm. The output files may all have the same prefix which may be the output file name (OutFileName) specified by the user. Examples of the output files are described below:

OutFileName.txt - A calls file containing the multiallelic calls of each sample for all the probeset groups. The calls file resembles the normal AxiomGT1.calls.txt file with sample names for the columns. However, the probeset_id column is populated by the probeset group id, which should be an id shared by two or more biallelic probesets. The calls are in the format AA to FF, which is the DMET calls format. For a given marker, the alleles that are interrogating that marker should be linked back lexographically to these symbols to map the actual alleles to the call. There are three other possible calls, which are no calls: NotAvailable - calls in which there was too much signal in all the other channels so the sample was not genotyped.

CallsInconsistent - there were two different calls that were equally called when biallelic calls were combined.

XX - a no call had been assigned during genotyping and combining genotype calls.

OutFileName_summary.txt - a summary file with channel signals converted to allele signals

OutFileName_ProbesetSummary.txt - data summarizing the probeset groups. The columns are as follows:

ps_group - contains the probeset group id

multi_asid - the multiallelic probeset id

multi_alleles - the alleles that are being interrogated for the marker

tile_strand - forward or reverse

line - identification to group wobbles (a probeset id either multiallelic or biallelic should be used in the future)

offset - the offset for this group of probesets

probeLength - the length of the probes in this group of probesets

nAllelesFound - the number of alleles that were actually found during the calling process

AllelesFound - which alleles were found of the expected alleles

nBiallelicCombinations - the number of biallelic combinations

AveBgnd - the average background value for this group of probesets

VarBgnd - the variance of the background for this group of probesets

WeightedAveBgnd - Weighted average background value, used to calculate background threshold

WeightedVarBgnd - Weighted standard deviation background value, used to calculate background threshold

SignalThreshold_weightedBNDPlus2sd - Background threshold. Value is used to determine if signal is above background

OutFileName_spotfire.txt- a file that summarizes all of the probeset groups and can be used to draw the clusters in spotfire. This is a file made for prototyping code and debugging. Some of the data may be desirable in the future for debugging purposes.

The following files are output from the assign_final_calls.py script:

calls file - file contains the final calls assigned from the multiallelic genotyping algorithm

confidences file - the confidences for the calls assigned

snp-posterior file - a file that contains the posterior parameters for the clusters which on subsequent runs can be used as a priors file

probabilities file - file contains the probabilities for a given sample belonging to each of the clusters

## MULTIALLELIC GENOTYPING - ALGORITHM VERIFICATION

In an example, an Axiom® array was designed with probesets for about 100,000 multiallelic markers each with more than two alleles in 1000 Genomes phase 3 data. Markers were selected for the presence of at least one example of the third-most common allele among the samples on the following four sample plates: T01 (CEU), T02 (CHB + JPT), T03 (YRI), and V12 (LWK) HapMap sample plates from Coriell. Probesets were mostly designed to avoid nearby, interfering variants (NIVs) with greater than 1% minor allele frequency in any continental population, except for a set of several thousand exon markers for which up to two NIVs were permitted. Probesets were tiled at two replicates, except for exon probesets which were tiled at four reps. Markers were selected from the autosomal chromosomes and chromosome X.

The four population plates listed above were assayed on the Axiom array plates. Sample quality control (QC) was performed as usual, employing the 3,000 AFFX-SNP biallelic control probesets to assess QC call rate. Signals from samples passing QC were used as input to the prototype implementation of the BANG algorithm. Generic priors and default algorithm parameters were used at each step. A probeset was considered to perform well if the probeset met the following criteria:

1. 90% call rate
2. Allele CN concordance > 50% for each allele copy number.

   a. Concordance to copy number 2 was calculated for each allele separately, as the number of correctly called homozygotes, divided by the expected number of homozygotes from the 1000 Genomes reference. No-calls were counted as errors.
   b. Concordance to copy number 1 was calculated for each allele separately, as the number of correctly called heterozygotes including the allele, divided by the expected number of heterozygotes including the allele. That is, CN 1 concordance for allele A was the fraction of correct calls among the samples with expected AB, AC, etc., genotypes. No-calls were counted as errors.

By these criteria, about 42% of probesets in the Axiom array performed well. Figure 9 illustrates an example plot of multiallelic probeset call rate versus an unweighted average of concordance to each expected genotype cluster. Concordance may be calculated for each expected genotype and then averaged without regard to the number of samples expected in each cluster. For example, expected genotypes may be AA, AB, AC, while there might not be expected samples with genotypes BB, CC, BC. Thus, those clusters may be omitted. No-calls assignments may be considered incorrect. All multiallelic probesets on the Axiom array are shown, genotyped on ~360 individuals from the CEU, CHB, JPT, YRI, and LWK populations. A large minority of probesets may have high call rate and concordance (top right corner of Figure 9). There may be another density peak at high call rate and low concordance, and probesets across the range of performance.

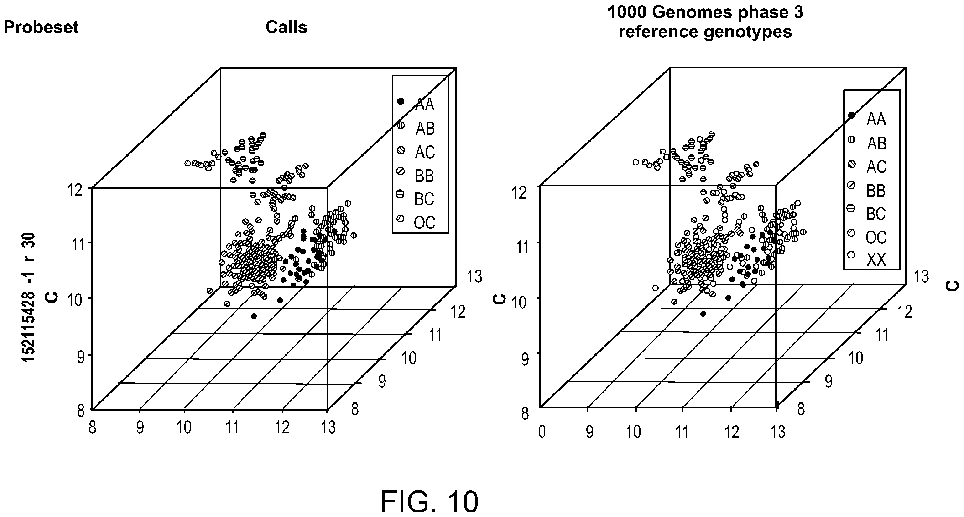

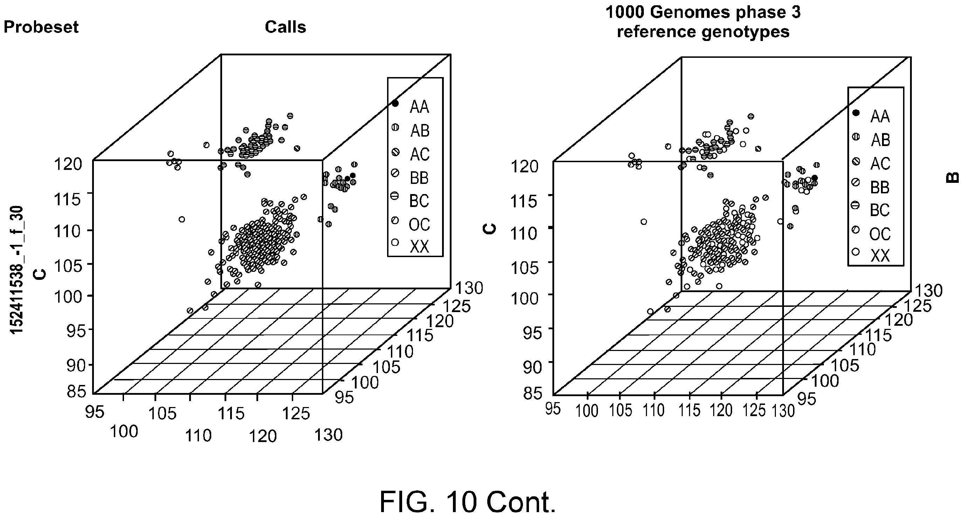

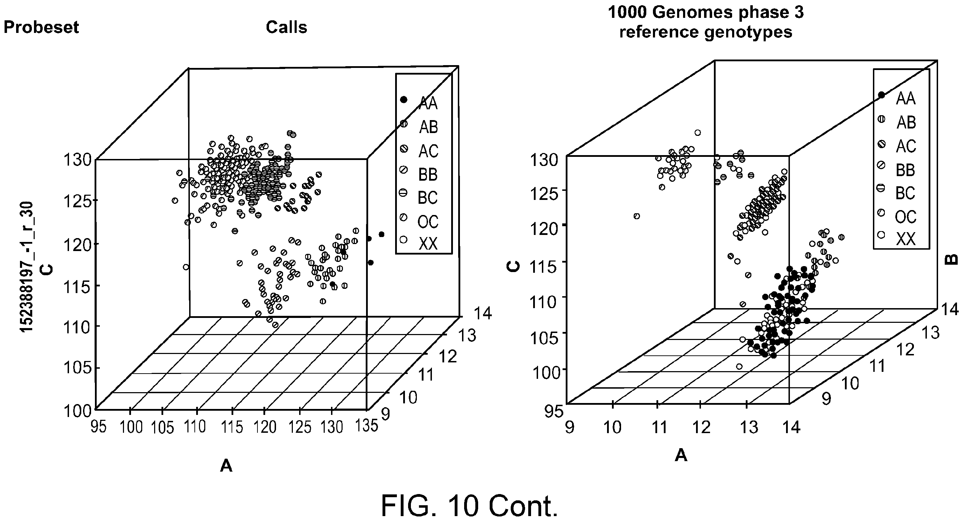

Figure 10 illustrates example plots of calls and reference genotypes for several converted probesets. For example, the plots in Figure 10 show BANG calls versus 1000 Genomes phase 3 reference genotypes. Three probesets for different tri-allelic markers are shown. All plots show log2 signals from the screening array on ~360 samples. Plots on the left are colored according to the genotypes assigned by the BANG algorithm. Plots on the right are colored according to the 1000 Genomes phase 3 calls for the same individuals. The larger number of no-calls (yellow) in the 1000 genomes reference genotypes reflects the fact that not all of the screened individuals were assayed by 1000 Genomes.

## PROBE DESIGN

Specific probe design may be related to multiallelic genotyping approaches and may be beneficial for obtaining data of interest. Logic routines for the determination of SNP probes that can be used in various DNA analysis systems have long existed. Previous arrays designed to interrogate SNPs would commonly utilize probe sets that contained a probe that was perfectly complementary to a target of interest (including the SNP of interest) and one or more other probes which contained one or more monosubstitutions as compared to the perfectly complementary probe. The resulting intensity data for the different probes in the probe set would then be compared to produce a genotype call for the SNP of interest. See, e.g., US Patent No. 5,858,659.

More recent arrays for genotyping SNPs include Axiom® Arrays (Affymetrix, Inc., Santa Clara, CA) and Infinium® II Arrays (Illumina, Inc., San Diego, CA). These arrays utilize a SNP probe that is complementary to a sequence that flanks the SNP site within the target nucleic acid of interest, and thus the SNP probe in these arrays does not directly hybridize with the target nucleic acid at the SNP site. Instead, the double-stranded portion of the probe-target duplex ends immediately upstream of the SNP. Interrogation of the SNP site is then accomplished by the addition of a nucleotide or probe (with the nucleotide or probe comprising one of two different haptens) to one end of the SNP probe (e.g., 5' ,3') through an appropriate mechanism known in the art that requires complementarity to the base of the target at the SNP site (e.g., ligation or single base extension). Determination of what allele was present at the SNP site is ascertained through subsequent detection of the particular hapten associated with the nucleotide or probe that was added.

The Axiom® Assay utilizes 30-base oligonucleotide SNP probes in a two color format. The identity of the base at the SNP site is ascertained by the ligation of probes containing one of two haptens that serve as attachment sites for one of two fluorescent labels, depending the identity of the base to the ligated to the SNP probe (e.g., a first hapten/label combination is associated with probes that will ligate when the SNP site is A or T, and a second hapten/label combination is associated with probes that will ligate when the SNP site is C or G). See, e.g., Hoffmann et al., "Next generation genome-wide association tool: design and coverage of a high-throughput European-optimized SNP array," Genomics, 98(2): 79-89 (2011); and Hoffmann et al., "Design and coverage of high throughput genotyping arrays optimized for individuals of East Asian, African American, and Latino race/ethnicity using imputation and a novel hybrid SNP selection algorithm," Genomics, 98(6): 422-30 (2011).

The Axiom® DMET assay may also be employed for genetic analysis of the involvement of metabolic pathways in drug metabolism. Genetic variation may be an important determinant in the ability of different individuals to metabolize drugs. Studies of an individual's genetic background may be used to target medications and to adjust treatment dose depending on the polymorphisms present in the individual. The DMET panel facilitates such testing by providing a single assay that analyzes more than 1,200 polymorphisms in a set of genes that may play a role in drug metabolism. The DMET panel may interrogate many different genes simultaneously, facilitating the detection of particular combinations of alleles in different genes that may be involved in the metabolism of a new drug.

The Infinium® II Assay utilizes 50-base oligonucleotide SNP probes in a two color format. The identity of the base at the SNP site is ascertained by the incorporation of ddNTPs bearing one of two different haptens through single base extension of the SNP probe, with each hapten associated with a different fluorescent label (e.g., ddCTP and ddGTP are associated with a first hapten/label combination while ddATP and ddTTP are associated with a second hapten/label combination). See, e.g., Gunderson et al., "Whole-genome genotyping of haplotype tag single nucleotide polymorphisms," Pharmacogenomics, 7(4): 641-8 (2006); and Steemers et al., "Whole-genome genotyping with the single-base extension assay," Nature Methods, 3: 31-33 (2006).

## COMBINED WHOLE GENOME AND LOCUS SPECIFIC AMPLIFICATION METHODS

In additional embodiments of the present disclosure, whole genome amplification (WGA) and locus-specific amplification may be combined for use with an array assay to selectively bias amplification toward desired target sequences in order to improve the resulting genotyping data for the desired target sequences and to reduce the effect of undesired pseudogenes in resulting data.

For example, the Axiom® and Infinium® II Assays utilize whole-genome amplified DNA, in which amplification is performed of an entire genome. Many whole genome amplification approaches are known in the art, such as Multiple Displacement Amplification (MDA), Degenerated Oligonucleotide PCR (DOP-PCR) and Primer Extension Preamplification (PEP), and many kits for WGA are commercially available, such as the PicoPLEX™ WGA Kit (New England Biolabs, Inc., Ipswich, Massachusetts), the REPLI-g WGA Kits (QIAGEN, Venlo, Netherlands) and the GenomePlex® Whole Genome Amplification Kits (Sigma-Aldrich Corporation, St. Louis, Missouri). For multiallelic targets, there may be an actual gene variant of interest, as well as pseudogenes that may be close in sequence but may still have minor differences from the actual variants of interest and which would not only not provide useful data to a particular clinical or research goal but indeed hinder efficient interrogation and genotyping of the target of interest. Pseudogenes may complicate the analysis of related sequences and may cause homozygous calls to appear as heterozygous calls or vice versa. Thus, whole genome amplification (WGA) may result in the amplification of such pseudogenes at a similar degree of amplification as the targets of interest, which may lead to difficulties in making accurate genotype calls for the targets of interest.

To overcome this, it may be beneficial to enhance results by supplementing whole genome amplification (WGA) with locus-specific amplification for the variants of interest. Many forms of locus specific amplification are known in the art, such as the use of multiplex polymerase chain reaction, molecular inversion probes with subsequent PCR, padlock probes with subsequent rolling circle amplification, and other approaches. Multiplex PCR may consist of using PCR to amplify different DNA target sequences simultaneously. That is, targets that contain pseudogenes may be subjected to mPCR amplification using primers that are specific to the desired gene. The use of molecular inversion probes is known in the art, and described in, e.g., Hardenbol et al., Nat. Biotechnol. 21:673-8 (2003), Hardenbol et al., Genome Res. 15:269-275 (2005), Ji et al., Cancer Res. 66:7910-9, U.S. Patent Nos. 6,858,412; 8,716,190; 8,828,688; 8,759,036, and U.S. Published Application Nos. 2013/0296172 and 2015/0284786. The use of padlock probes with subsequent rolling circle amplification is also known in the art, and described in, e.g., U.S. Patent Nos. 6,558,928 and 7,074,564. Locus-specific amplification of one particular target sequence (instead of its similar variants) may be helpful with subsequent analysis of data obtained from a microarray assay, particularly when combined with whole genome amplification.

Locus-specific amplification can be utilized to supplement whole genome amplification in order to ultimately create more copies of the desired targets of the genome and statistically bias the resulting amplification products toward the desired targets as opposed to undesired pseudogenes. Increasing the concentration of the target section of the genome relative to the undesired regions can increase the signal from the target and enhance the subsequent genotyping results in both biallelic and multiallelic genotyping. In other words, increasing the desired amplicons available for interrogation on the array may lead to a more efficient and enhanced bioinformatics genotyping process. The improvement of the genotyping process can be particularly beneficial when a particular target of marker of interest, such as a specific SNP in a gene variant of interest, has many similar pseudogenes or variants. For example, within cytochrome P450, there are many markers of diagnostic, clinical, and/or pharmacogenomics significance, but that have close variants that are not relevant. For example, there are SNPs within CYP2D6 that are of high pharmacogenomics value, but relying on whole genome amplification alone may hinder the accurate analysis of the desired marker as high homology between CYP2D6 and pseudogenes such as CYP2D7 and CYP2D8 will commonly complicate the subsequent genotyping through the latter's contribution of a high non-specific background signal to the interrogation of the CYP2D6 markers (e.g., SNPs) of interest. As would be recognized by one of skill in the art, this high non-specific background from pseudogenes and otherwise sequences of high homology also hinders other markers of interest, such as ABCC2, CFTR, CYP1A2, CYP2A6, CYP2B6, CYP2C19, CYP2C8, CYP2C9, GSTM1, and SULT1A1.

In some embodiments, a genomic DNA sample may be obtained (such as by extraction) and whole genome amplification may be applied to the sample. Locus-specific mPCR amplification may be performed on the sample to target sequences of interest, and the sample may be fragmented and hybridized to an array for multiallelic genotyping.

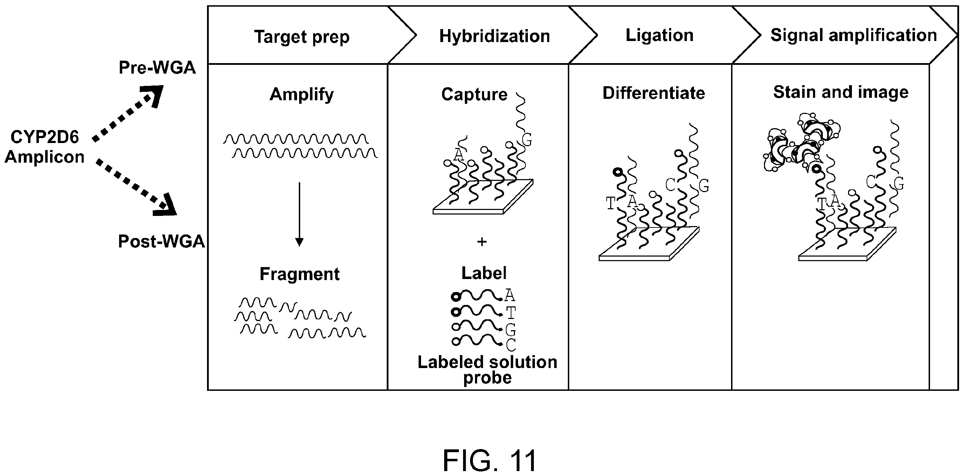

Figure 11 illustrates an example diagram of a flow of steps in the disclosed amplification approach in accordance with one or more aspects of the present disclosure. In this example, a CYP2D6 5.6 kb PCR product may be added to an Axiom® workflow at two different steps: before whole genome amplification or after whole genome amplification. In some embodiments, this workflow may allow ~100 variants (2,973 probesets) to be looked at using a single PCR product.

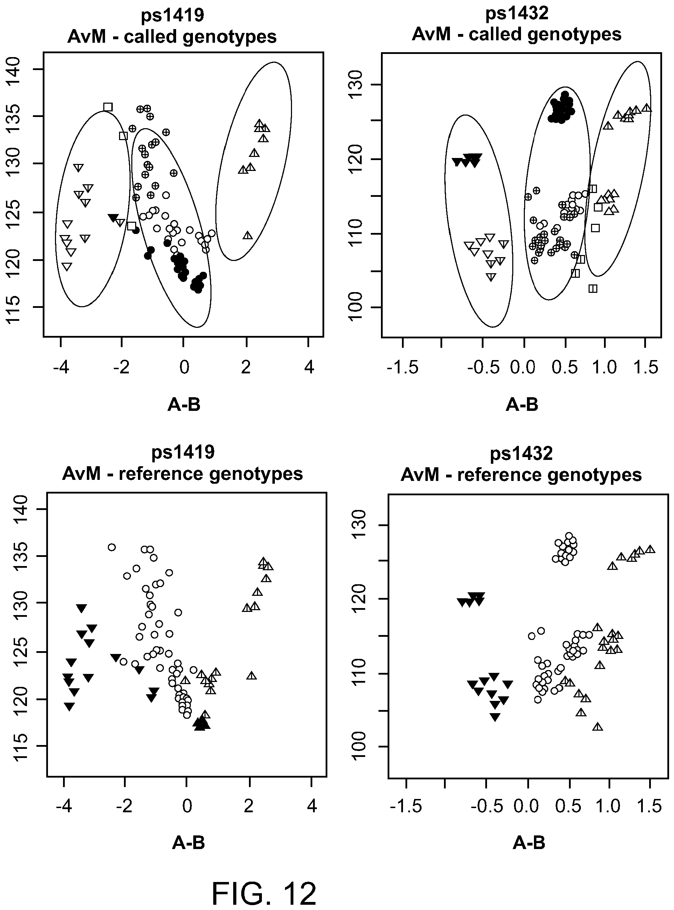

Figure 12 illustrates plots of results obtained from testing two procedures for using PCR-amplified targets. The plots in Figure 12 show cluster plots obtained from amplifying a single amplicon including all of CYP2D6. In some embodiments, the procedures for using PCR-amplified targets may result in a slightly improved SNP conversion rate. However, a larger study may be necessary to evaluate impact on difficult markers.

In some embodiments, the Axiom® DMET amplification methods disclosed herein may take advantage of the Axiom 2.0 robust chemistry platform with a similar workflow, with a 24-well format, manual target preparation, and reagent handling. The mPCR step may be incorporated into Axiom® workflow using commercially available mPCR kits, such as the QIAGEN Multiplex PCR Kit. In some embodiments, mPCR products may be added post-whole genome amplification, prior to DNase fragmentation in the workflow.

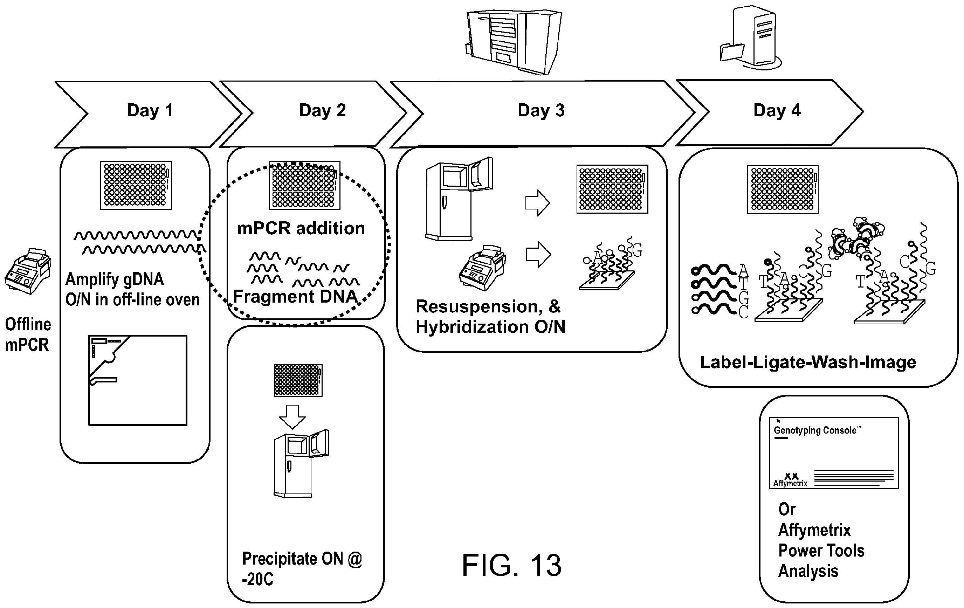

Figure 13 illustrates an example diagram of the workflow in the disclosed amplification approach in accordance with one or more aspects of the present disclosure.

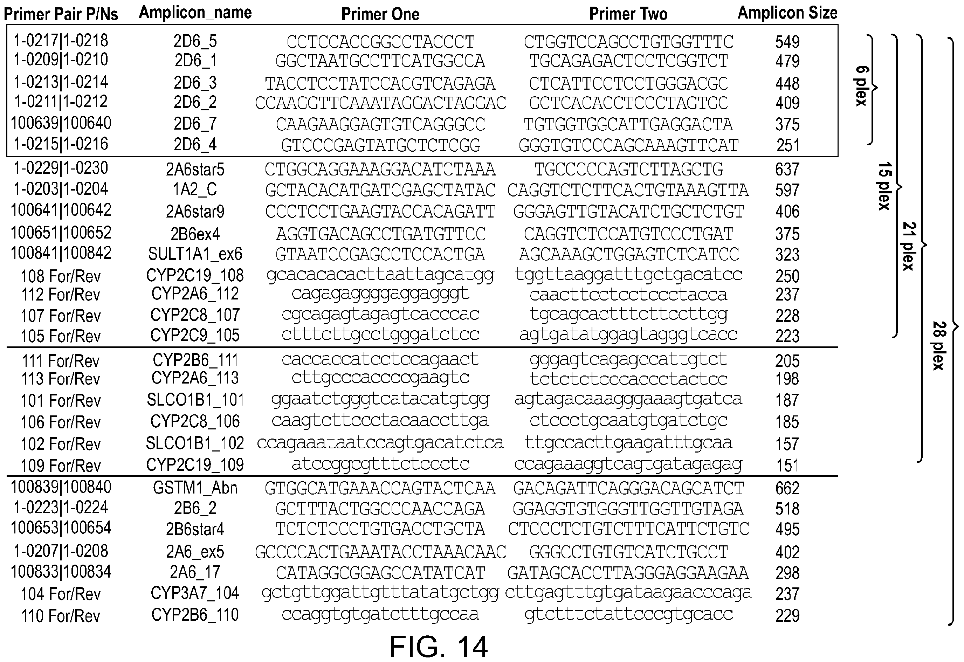

Figure 14 illustrates a table of multiplexing primer sets tested for feasibility in accordance with one or more aspects of the present disclosure.

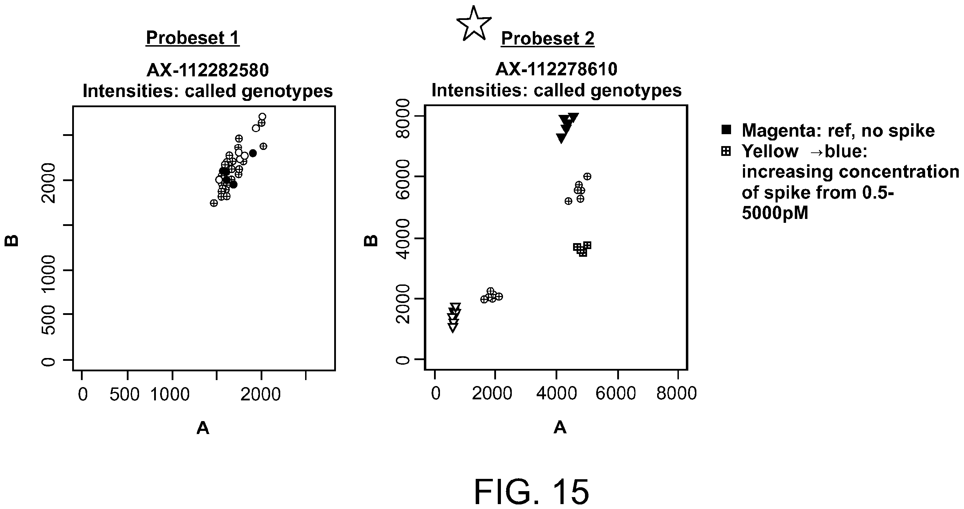

Additionally, oligonucleotide spike-in studies may help identify responsive probes. In one example, 70-mer oligomers were synthesized for Tier 1 monomorphs (e.g., A, B, C, D alleles). The oligomers match both strands and have sequence degeneracy at wobble positions. The amplified gDNA was processed on the DMET array plate and probe response was monitored. Figure 15 illustrates an example of genotyping results from the spike-in studies. As shown in Figure 15, the first probeset was unresponsive, whereas the second probeset showed dose-dependent response.

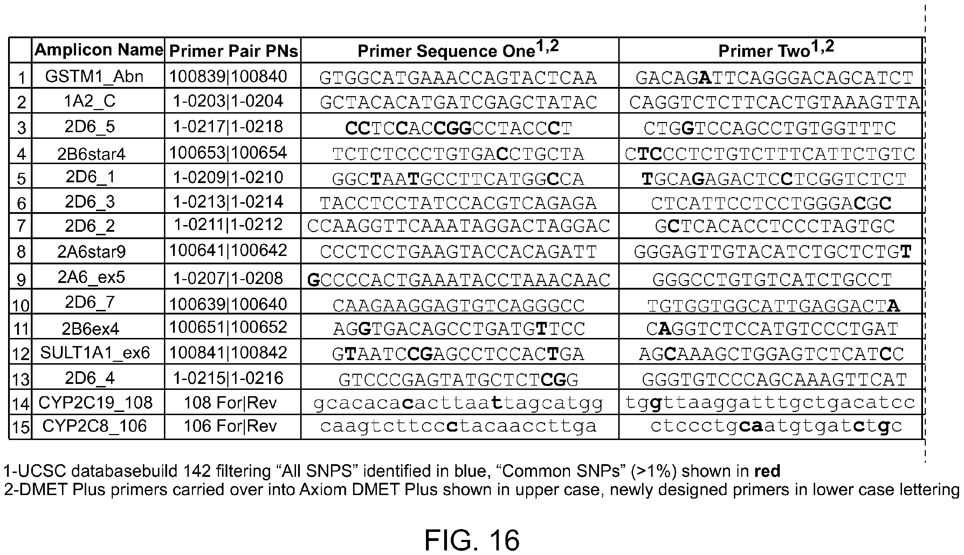

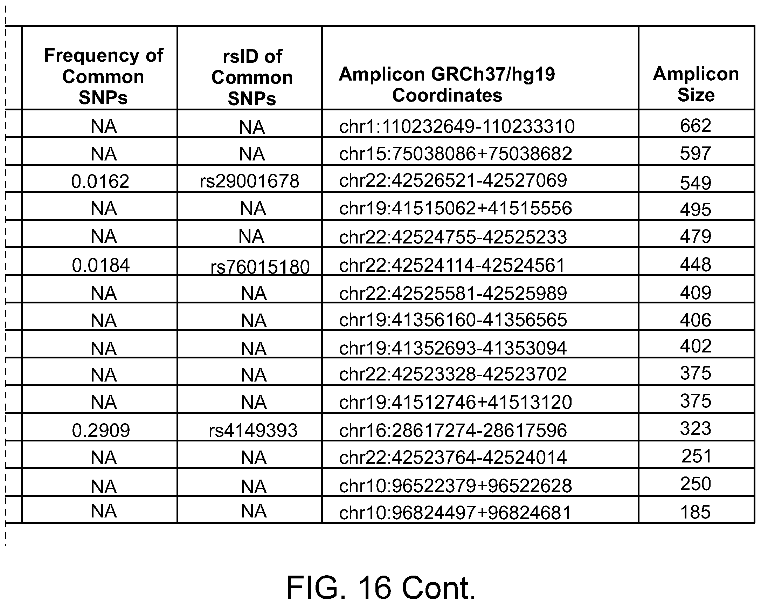

Figure 16 illustrates an example table of results from a 15-plex mPCR assay in accordance with one or more aspects of the present disclosure. In some embodiments, a Qiagen Multiplex PCR Plus Kit (PN 206151 or 206152) may be utilized in an mPCR protocol. As indicated in the results shown in Figure 16, three SNPs with a minor allele frequency (MAF) $\geq$ 1% were observed in primer sequences carried over from DMET Plus. Reference SNP variant rs76015180 was present at a critical 3' end of 1-0214 primer and was shown to affect amplification.

Although the subject matter has been described in language specific to structural features and/or methodological acts', it is to be understood that the subject matter defined in the appended claims is not necessarily limited to the specific features or acts described above. Rather, the specific features and acts described above are described as example implementations of the following claims.

**Claims**

1. A method for genotyping one or more multiallelic markers using a computer system, the method comprising:

   acquiring signals for one or more multiallelic markers in one or more samples, said one or more multiallelic markers comprising n alleles, wherein n is the number of possible alleles that is three or more;
   for each multiallelic marker, clustering the signals for each pair of alleles in a plurality of allele pairs from the one or more samples, resulting in clusters representing each allele pair;
   for each homozygous cluster representing a homozygous allele pair, collecting signals for an alternative allele for calculation of a background signal for the alternative allele, resulting in a plurality of background signals each representing a respective allele;
   assigning initial genotype calls for each sample for each allele pair based upon the signals and the background signals;
   calculating a multivariate normal distribution in an n-dimensional space for each cluster using the initial genotype calls and prior cluster parameters;
   for each multivariate normal distribution for each cluster, calculating a logarithmic likelihood of membership for each sample;
   based on the logarithmic likelihood of membership, calculating, for each sample, a probability of membership in each cluster; and
   assigning a final genotype call to each sample based on the probability of membership.

2. The method of claim 1, wherein the one or more multiallelic markers comprise single nucleotide polymorphisms (SNPs) and indels.

3. The method of claim 1, wherein the signals acquired for the one or more multiallelic markers comprise allele intensity data for each allele in the one or more samples.

4. The method of claim 1, wherein clustering the signals further comprises using an algorithm with defined single nucleotide polymorphisms (SNPs) and defined algorithm parameters.

5. The method of claim 1, wherein assigning the initial genotype calls further comprises:

   for each allele pair, identifying a subset of samples that do not have a signal above the background signal in any alternative allele;
   for each allele pair, determining that the number of samples in the subset of samples is above a predefined minimum value; and
   for each allele pair, genotyping each sample in the subset of samples for the two alleles represented in the corresponding allele pair;

6. The method of claim 1, wherein assigning the initial genotype calls for each sample further comprises:
   comparing calls for each sample in order to choose a call occurring most frequently in each sample, wherein if there is a tie among the calls, a "no-call" value is assigned to the sample.

7. The method of claim 1, wherein calculating the logarithmic likelihood of membership for each sample is calculated using

$$\ln(L) = -\ln(|\Sigma|) - \frac{1}{2}(x - \mu)^T \Sigma^{-1}(x - \mu) - \frac{k}{2}\ln(2\pi)$$

   wherein $|\Sigma|$ *is the determinant of covariance;*
   wherein x is the k-dimensional column vector containing the signal for a sample for a probe set;
   wherein k is the number of signals for a probe set.

8. The method of claim 1, wherein assigning the final genotype call further comprises:

assigning each sample to a particular cluster for which the sample has the highest probability of membership, resulting in a cluster assignment for each sample; and
assigning the final genotype call based on the cluster assignment for each sample.

9. The method of claim 1, further comprising:

calculating a confidence value for each sample, wherein the confidence value comprises a probability of membership of the sample in any other cluster;
comparing the confidence value for each sample with a predefined threshold value; and
assigning a "no-call" value to each sample that has a confidence value above the predefined threshold value.

10. The method of claim 1, further comprising:
calculating a mean, variance, and standard deviation of the background signal for each respective allele.

11. The method of claim 10, wherein a global estimated background signal is used for an allele if no values are available to calculate the mean, variance, and standard deviation of the background signal for the allele, and wherein the global estimated background signal is an average of the plurality of background signals.

12. The method of claim 10, wherein calculating the mean, variance, and standard deviation of the background signal for each respective allele, further comprises calculating using the following equations:

$$avgSig_{allele} = \sum allele_{hom} / nsig_{allele}$$

$$bgnd_{allele} = \sum allele_{in\,hom\,call\,not=allele}$$

$$avgBgnd_{allele} = bgnd_{allele} / nsig_{allele}$$

$$weightedAvgBgnd = \sum(avgBgnd_{allele} * nsig_{allele}) / \sum nsig_{allele}$$

$$varianceBgnd_{allele} = \sum(bgnd_{allele\,i} - avgBgnd_{allele})^2 / nsig_{allele} - 1$$

$$stdevBgnd_{allele} = \sqrt{varianceBgnd_{allele}}$$

$$weightedAvgStDevBgnd = \sum(stdevBgnd_{allele} * nsig_{allele}) / \sum nsig_{allele}$$

wherein $avgSig_{allele}$ is the average signal for an allele, $allele_{hom}$ is the signal of homozygote calls for that allele, $nsig_{allele}$ is the total number of samples that contributed to the signals;
$bgnd_{allele}$ is the background value of an allele, $\sum allele_{in\,hom\,call\,not=allele)}$ is the signal for that allele during homozygote calls when the call does not match the allele;
$avgBgnd_{allele}$ is the average for the background of an allele;
$weightedAvgBgnd$ is the weighted average of the background;
$varianceBgnd_{allele}$ is the variance of the background;
$stdevBgnd_{allele}$ = is the standard deviation of the background; and
$weightedAvgStDevBgnd$ is the weighted average standard deviation of the background.

13. The method of claim 1, wherein acquiring the signals for the one or more multiallelic markers in the one or more samples is based on hybridization of the samples with a plurality of probes on an array for measuring the multiallelic markers.

14. The method of claim 13, further comprising:
determining an allele present in a target sequence of the sample by ligating differentially labeled oligonucleotides to the plurality of probes on the array to distinguish between ligation of labeled oligonucleotides with A, T, C, or G nucleotides at the 3' end of the labeled oligonucleotides.

15. The method of claim 13, further comprising:
determining an allele present in a target sequence of the sample by using single base extension of the plurality of the probes on the array with differentially labeled nucleotides to distinguish between extension with A, T, C, or G nucleotides.

**Patentansprüche**

1. Verfahren zum Genotypisieren eines oder mehrerer multiallelischer Marker unter Verwendung eines Computersystems, wobei das Verfahren Folgendes umfasst:

Erfassen von Signalen für einen oder mehrere multiallelische Marker in einer oder mehreren Proben, wobei der eine oder die mehreren multiallelischen Marker n Allele umfassen, wobei n die Anzahl der möglichen Allele ist, die drei oder mehr beträgt;
für jeden multiallelischen Marker, Clustern der Signale für jedes Paar von Allelen in mehreren Allelpaaren aus der einen oder den mehreren Proben, was zu Clustern führt, die jedes Allelpaar repräsentieren;
für jeden homozygoten Cluster, der ein homozygotes Allelpaar repräsentiert, Sammeln von Signalen für ein alternatives Allel für eine Berechnung eines Hintergrundsignals für das alternative Allel, was zu mehreren Hintergrundsignalen führt, von denen jedes ein jeweiliges Allel repräsentiert;
Zuweisen von anfänglichen Genotyp-Calls für jede Probe für jedes Allelpaar basierend auf den Signalen und den Hintergrundsignalen;
Berechnen einer multivariaten Normalverteilung in einem n-dimensionalen Raum für jeden Cluster unter Verwendung der anfänglichen Genotyp-Calls und vorheriger Cluster-Parameter;
für jede multivariate Normalverteilung für jeden Cluster, Berechnen einer logarithmischen Wahrscheinlichkeit einer Zugehörigkeit für jede Probe;
basierend auf der logarithmischen Wahrscheinlichkeit der Zugehörigkeit, Berechnen, für jede Probe, einer Wahrscheinlichkeit der Zugehörigkeit zu jedem Cluster; und
Zuweisen eines endgültigen Genotyp-Calls zu jeder Probe basierend auf der Wahrscheinlichkeit der Zugehörigkeit.

2. Verfahren nach Anspruch 1, wobei der eine oder die mehreren multiallelischen Marker Einzelnukleotid-Polymorphismen (*single nucleotide polymorphisms* - SNPs) und Indels umfassen.

3. Verfahren nach Anspruch 1, wobei die für den einen oder die mehreren multiallelischen Marker erfassten Signale Allelintensitätsdaten für jedes Allel in der einen oder den mehreren Proben umfassen.

4. Verfahren nach Anspruch 1, wobei das Clustern der Signale ferner ein Verwenden eines Algorithmus mit definierten Einzelnukleotid-Polymorphismen (SNPs) und definierten Algorithmus-Parametern umfasst.

5. Verfahren nach Anspruch 1, wobei das Zuweisen der anfänglichen Genotyp-Calls ferner Folgendes umfasst:

für jedes Allelpaar, Identifizieren einer Untergruppe von Proben, die bei keinem alternativen Allel ein Signal über dem Hintergrundsignal aufweisen;
für jedes Allelpaar, Bestimmen, dass die Anzahl der Proben in der Untergruppe der Proben über einem vordefinierten Mindestwert liegt; und
für jedes Allelpaar, Genotypisieren jeder Probe in der Untergruppe von Proben für die zwei Allele, die in dem entsprechenden Allelpaar repräsentiert sind;

6. Verfahren nach Anspruch 1, wobei das Zuweisen der anfänglichen Genotyp-Calls für jede Probe ferner Folgendes umfasst:
Vergleichen von Calls für jede Probe, um einen Call auszuwählen, der in jeder Probe am häufigsten vorkommt, wobei, falls ein Gleichstand unter den Calls vorliegt, ein "Nicht-Call"-Wert der Probe zugewiesen wird.

7. Verfahren nach Anspruch 1, wobei das Berechnen der logarithmischen Wahrscheinlichkeit der Zugehörigkeit für jede Probe unter Verwendung von Folgendem berechnet wird:

$$\ln(L) = -\ln(|\Sigma|) - \frac{1}{2}(x - \mu)^T \Sigma^{-1}(x - \mu) - \frac{k}{2}\ln(2\pi)$$

wobei $|\Sigma|$ *die Determinante der Kovarianz ist;*
wobei x der k-dimensionale Spaltenvektor ist, der das Signal für eine Probe für einen Sondensatz enthält;
wobei k die Anzahl der Signale für einen Sondensatz ist.

8. Verfahren nach Anspruch 1, wobei das Zuweisen des endgültigen Genotyp-Calls ferner Folgendes umfasst:

Zuweisen jeder Probe zu einem bestimmten Cluster, für den die Probe die höchste Wahrscheinlichkeit der Zugehörigkeit aufweist, was zu einer Cluster-Zuweisung für jede Probe führt; und
Zuweisen des endgültigen Genotyp-Calls basierend auf der Cluster-Zuweisung für jede Probe.

9. Verfahren nach Anspruch 1, ferner Folgendes umfassend:

Berechnen eines Konfidenzwertes für jede Probe, wobei der Konfidenzwert eine Wahrscheinlichkeit der Zugehörigkeit der Probe zu einem beliebigen anderen Cluster umfasst;
Vergleichen des Konfidenzwertes für jede Probe mit einem vordefinierten Schwellenwert; und
Zuweisen eines "Nicht-Call"-Wertes zu jeder Probe, die einen Konfidenzwert über dem vordefinierten Schwellenwert aufweist.

10. Verfahren nach Anspruch 1, ferner Folgendes umfassend:
Berechnen eines arithmetischen Mittels, einer Varianz und einer Standardabweichung des Hintergrundsignals für jedes jeweilige Allel.

11. Verfahren nach Anspruch 10, wobei ein global geschätztes Hintergrundsignal für ein Allel verwendet wird, falls keine Werte verfügbar sind, um das arithmetische Mittel, die Varianz und die Standardabweichung des Hintergrundsignals für das Allel zu berechnen, und wobei das global geschätzte Hintergrundsignal ein Durchschnitt der mehreren Hintergrundsignale ist.

12. Verfahren nach Anspruch 10, wobei das Berechnen des arithmetischen Mittels, der Varianz und der Standardabweichung des Hintergrundsignals für jedes jeweilige Allel ferner das Berechnen unter Verwendung der folgenden Gleichungen umfasst:

$$avgSig_{allele} = \sum allele_{hom}/nsig_{allele}$$

$$bgnd_{allele} = \sum allele_{in\ hom\ call\ not\ =allele}$$

$$avgBgnd_{allele} = bgnd_{allele}/nsig_{allele}$$

$$weightedAvgBgnd = \sum(avgBgnd_{allele} * nsig_{allele})/\sum nsig_{allele}$$

$$variceBgnd_{allele} = \sum \left(bgnd_{allele_i} - avgBgnd_{allele}\right)^2 / nsig_{allele} - 1$$

$$stdevBgnd_{allele} = \sqrt{varianceBgnd_{allele}}$$

$$weightedAvgStDevBgnd = \sum \left(stdevBgnd_{allele} * nsig_{allele}\right) / \sum nsig_{allele}$$

wobei $avgSig_{allele}$ das durchschnittliche Signal (*average Signal*) für ein Allel (*allele*) ist, $allele_{hom}$ das Signal der homozygoten Calls für jenes Allel ist, $nsig_{allele}$ die Gesamtanzahl der Proben ist, die zu den Signalen beigetragen haben;

$bgnd_{allele}$ der Hintergrundwert (*background value*) eines Allels ist, $\sum allele_{in\ hom\ call\ not=allele}$). das Signal für jenes Allel während homozygoter Calls ist, wenn der Call nicht mit dem Allel übereinstimmt;

$avgBgnd_{allele}$ der Durchschnitt (*average*) für den Hintergrund (*background*) eines Allels ist;

*weightedAvgBgnd* der gewichtete Durchschnitt (*weighted average*) des Hintergrunds ist;

$varianceBgnd_{allele}$ die Varianz (*variance*) des Hintergrunds ist;

$stdevBgnd_{allele}$ = die Standardabweichung (*standard deviation*) des Hintergrunds ist; und

*weightedAvgStDevBgnd* die gewichtete durchschnittliche Standardabweichung (*weighted average standard deviation*) des Hintergrunds ist.

**13.** Verfahren nach Anspruch 1, wobei das Erfassen der Signale für den einen oder die mehreren multiallelischen Marker in der einen oder den mehreren Proben auf einer Hybridisierung der Proben mit mehreren Sonden auf einem Array zum Messen der multiallelischen Marker basiert.

**14.** Verfahren nach Anspruch 13, ferner Folgendes umfassend:
Bestimmen eines in einer Zielsequenz der Probe vorhandenen Allels durch Ligieren differentiell markierter Oligonukleotide an die mehreren Sonden auf dem Array, um zwischen einer Ligation markierter Oligonukleotide mit A-, T-, C- oder G-Nukleotiden an dem 3'-Ende der markierten Oligonukleotide zu unterscheiden.

**15.** Verfahren nach Anspruch 13, ferner Folgendes umfassend:
Bestimmen eines in einer Zielsequenz der Probe vorhandenen Allels durch Verwenden einer Einzelbasenverlängerung der mehreren Sonden auf dem Array mit unterschiedlich markierten Nukleotiden, um zwischen der Verlängerung mit A-, T-, C- oder G-Nukleotiden zu unterscheiden.

## Revendications

**1.** Procédé de génotypage d'un ou plusieurs marqueurs multialléliques à l'aide d'un système informatique, le procédé comprenant :

l'acquisition des signaux pour un ou plusieurs marqueurs multialléliques dans un ou plusieurs échantillons, lesdits un ou plusieurs marqueurs multialléliques comprenant n allèles, n étant le nombre d'allèles possibles qui est de trois ou plus ;

pour chaque marqueur multiallélique, le regroupement des signaux pour chaque paire d'allèles dans une pluralité de paires d'allèles à partir du ou des échantillons, afin d'obtenir des groupes représentant chaque paire d'allèles ;

pour chaque groupe homozygote représentant une paire d'allèles homozygote, la collection des signaux pour un allèle alternatif pour le calcul d'un signal de fond pour l'allèle alternatif, afin d'obtenir une pluralité de signaux de fond représentant chacun un allèle respectif ;

l'attribution de déterminations initiales de génotypes pour chaque échantillon pour chaque paire d'allèles en fonction des signaux et des signaux de fond ;

le calcul d'une distribution normale multidimensionnelle dans un espace n-dimensionnel pour chaque groupe à l'aide des déterminations initiales de génotypes et des paramètres de groupe précédents ;

pour chaque distribution normale multidimensionnelle pour chaque groupe, le calcul d'une probabilité logarithmique d'appartenance pour chaque échantillon ;

en fonction de la probabilité logarithmique d'appartenance, le calcul, pour chaque échantillon, d'une probabilité d'appartenance dans chaque groupe ; et

l'attribution d'une détermination finale de génotype pour chaque échantillon en fonction de la probabilité d'appartenance.

2. Procédé selon la revendication 1, dans lequel le ou les marqueurs multialléliques comprennent des polymorphismes d'un seul nucléotide (PSN) et des indels.

3. Procédé selon la revendication 1, dans lequel les signaux acquis pour le ou les marqueurs multialléliques comprennent des données sur l'intensité des allèles pour chaque allèle dans le ou les échantillons.

4. Procédé selon la revendication 1, dans lequel le regroupement des signaux comprend en outre l'utilisation d'un algorithme avec des polymorphismes d'un seul nucléotide (PSN) définis et des paramètres d'algorithme définis.

5. Procédé selon la revendication 1, dans lequel l'attribution des déterminations initiales de génotypes comprend en outre :

pour chaque paire d'allèles, l'identification d'un sous-ensemble d'échantillons qui ne présente pas de signal au-dessus du signal de fond dans aucun allèle alternatif ;

pour chaque paire d'allèles, la détermination du fait que le nombre d'échantillons dans le sous-ensemble d'échantillons est supérieur à une valeur minimale prédéfinie ; et

pour chaque paire d'allèles, le génotypage de chaque échantillon dans le sous-ensemble d'échantillons pour les deux allèles représentés dans la paire d'allèles correspondante ;

6. Procédé selon la revendication 1, dans lequel l'attribution des déterminations initiales de génotypes pour chaque échantillon comprend en outre :

la comparaison des déterminations pour chaque échantillon afin de choisir une détermination se produisant le plus fréquemment dans chaque échantillon, en cas de correspondance entre les déterminations, une valeur de « non-détermination » étant attribuée à l'échantillon.

7. Procédé selon la revendication 1, dans lequel le calcul de la probabilité logarithmique d'appartenance pour chaque échantillon est calculé à l'aide de

$$\ln(L) = -\ln(|\textstyle\sum|) - \frac{1}{2}(x - \mu)^T \textstyle\sum^{-1}(x - \mu) - \frac{k}{2}\ln(2\pi)$$

$|\Sigma|$ *étant le déterminant de la covariance ;*

x étant le vecteur colonne k-dimensionnel contenant le signal pour un échantillon pour un ensemble de sondes ;

k étant le nombre de signaux pour un ensemble de sondes.

8. Procédé selon la revendication 1, dans lequel l'attribution de la détermination finale de génotype comprend en outre :

l'attribution de chaque échantillon à un groupe particulier pour lequel l'échantillon a la probabilité d'appartenance la plus élevée, afin d'obtenir une attribution de groupe pour chaque échantillon ; et

l'attribution de la détermination finale de génotype en fonction de l'attribution de groupe pour chaque échantillon.

9. Procédé selon la revendication 1, comprenant en outre :

le calcul d'un degré de confiance pour chaque échantillon, le degré de confiance comprenant une probabilité d'appartenance à l'échantillon de tout autre groupe ;

la comparaison du degré de confiance pour chaque échantillon à une valeur de seuil prédéfinie ; et

l'attribution d'une valeur de « non-détermination » à chaque échantillon ayant un degré de confiance supérieur à la valeur de seuil prédéfinie.

10. Procédé selon la revendication 1, comprenant en outre :

le calcul d'une moyenne, d'une variance et d'une déviation standard du signal de fond pour chaque allèle respectif.

11. Procédé selon la revendication 10, dans lequel un signal de fond général estimé est utilisé pour un allèle si aucune valeur n'est disponible pour calculer la moyenne, la variance et la déviation standard du signal de fond pour l'allèle, et dans lequel le signal de fond général estimé est une moyenne de la pluralité de signaux de fond.

12. Procédé selon la revendication 10, dans lequel le calcul de la moyenne, de la variance et de la déviation standard du signal de fond pour chaque allèle respectif, comprend en outre le calcul à l'aide des équations suivantes :

$$avgSig_{allele} = \sum allele_{hom}/nsig_{allele}$$

$$bgnd_{allele} = \sum allele_{in\ hom\ call\ not=allele}$$

$$avgBgnd_{allele} = bgnd_{allele}/nsig_{allele}$$

$$weightedAvgBgnd = \sum(avgBgnd_{allele} * nsig_{allele})/\sum nsig_{allele}$$

$$varianceBgnd_{allele} = \sum\left(bgnd_{allele_i} - avgBgnd_{allele}\right)^2/nsig_{allele} - 1$$

$$stdevBgnd_{allele} = \sqrt{varianceBgnd_{allele}}$$

$$weightedAvgStDevBgnd = \sum(stdevBgnd_{allele} * nsig_{allele})/\sum nsig_{allele}$$

$avgSig_{allele}$ étant le signal moyen pour un allèle, $allele_{hom}$ est le signal des déterminations homozygotes pour cet allèle, $nsig_{allele}$ est le nombre total d'échantillons ayant contribués aux signaux ;
$bgnd_{allele}$ est la valeur de fond d'un allèle, $\sum allele_{in\ hom\ call\ not=allele}$ est le signal de cet allèle pendant les déterminations homozygotes lorsqu'une détermination ne correspond pas à l'allèle ;
$avgBgnd_{allele}$ est la moyenne pour le fond d'un allèle ;
$weightedAvgBgnd$ est la moyenne pondérée du fond ;
$varianceBgnd_{allele}$ est la variance du fond ;
$stdevBgnd_{allele}$ = est la déviation standard du fond ; et
$weightedAvgStDevBgnd$ est la déviation standard de la moyenne pondérée du fond.

13. Procédé selon la revendication 1, dans lequel l'acquisition des signaux pour le ou les marqueurs multialléliques dans le ou les échantillons est basée sur l'hybridation des échantillons avec une pluralité de sondes sur un réseau pour mesurer les marqueurs multialléliques.

14. Procédé selon la revendication 13, comprenant en outre :
la détermination d'un allèle présent dans une séquence cible de l'échantillon en ligaturant des oligonucléotides marqués de manière différentielle à la pluralité de sondes sur le réseau pour faire la distinction entre la ligature des oligonucléotides marqués et les nucléotides A, T, C ou G à l'extrémité 3' des oligonucléotides marqués.

15. Procédé selon la revendication 13, comprenant en outre :
la détermination d'un allèle présent dans une séquence cible de l'échantillon à l'aide d'une extension à base unique de la pluralité de sondes sur le réseau avec des nucléotides marqués de manière différentielle pour faire la distinction entre l'extension et les nucléotides A, T, C ou G.

FIG. 1

EP 3 362 580 B1

| PROCESSOR | MEMORY | FIXED STORAGE | REMOVABLE STORAGE |
|---|---|---|---|
| 51 | 53 | 55 | 57 |

67

| DISPLAY ADAPTER | | SOUND CARD |
|---|---|---|
| 59 | | 61 |

| DISPLAY | KEYBOARD | MOUSE | SPEAKERS | NETWORK INTERFACE |
|---|---|---|---|---|
| 3 | 9 | 11 | 63 | 65 |

FIG. 2

FIG. 3

FIG. 4

Collecting signal data for all
samples

Estimating background signals
for samples

Selecting samples for biallelic
genotyping

Fitting samples to clusters

Genotyping samples

FIG. 5

FIG. 6C

FIG. 6B

FIG. 6A

FIG. 7C

FIG. 7B

FIG. 7A

FIG. 8

**Concordance vs Call Rate**

FIG. 9

FIG. 10

**Probeset**         **Calls**

**1000 Genomes phase 3 reference genotypes**

FIG. 10 Cont.

**Probeset**                    **Calls**

1000 Genomes phase 3
reference genotypes

EP 3 362 580 B1

FIG. 10 Cont.

FIG. 11

FIG. 12

Offline mPCR

**Day 1**

Amplify gDNA O/N in off-line oven

**Day 2**

mPCR addition

Fragment DNA

Precipitate ON @ -20C

**Day 3**

Resuspension, & Hybridization O/N

**Day 4**

Label-Ligate-Wash-Image

Genotyping Console™

Affymetrix

Or Affymetrix Power Tools Analysis

FIG. 13

EP 3 362 580 B1

| Primer Pair P/Ns | Amplicon_name | Primer One | Primer Two | Amplicon Size |
|---|---|---|---|---|
| 1-0217\|1-0218 | 2D6_5 | CCTCCACCGGCCTACCCT | CTGGTCCAGCCTGTGGTTTC | 549 |
| 1-0209\|1-0210 | 2D6_1 | GGCTAATGCCTTCATGGCCA | TGCAGAGACTCCTCGGTCT | 479 |
| 1-0213\|1-0214 | 2D6_3 | TACCTCCTATCCACGTCAGAGA | CTCATTCCTCCTGGGACGC | 448 |
| 1-0211\|1-0212 | 2D6_2 | CCAAGGTTCAAATAGGACTAGGAC | GCTCACACCTCCCTAGTGC | 409 |
| 100639\|100640 | 2D6_7 | CAAGAAGGAGTGTCAGGGCC | TGTGGTGGCATTGAGGACTA | 375 |
| 1-0215\|1-0216 | 2D6_4 | GTCCCGAGTATGCTCTCGG | GGGTGTCCCAGCAAAGTTCAT | 251 |
| 1-0229\|1-0230 | 2A6star5 | CTGGCAGGAAAGGACATCTAAA | TGCCCCAGTCTTAGCTG | 637 |
| 1-0203\|1-0204 | 1A2_C | GCTACACATGATCGAGCTATAC | CAGGTCTCTTCACTGTAAAGTTA | 597 |
| 100641\|100642 | 2A6star9 | CCCTCCTGAAGTACCACAGATT | GGGAGTTGTACATCTGCTCTGT | 406 |
| 100651\|100652 | 2B6ex4 | AGGTGACAGCCTGATGTTCC | CAGGTCTCCATGTCCCTGAT | 375 |
| 100841\|100842 | SULT1A1_ex6 | GTAATCCGAGCCTCCACTGA | AGCAAAGCTGGAGTCTCATCC | 323 |
| 108 For/Rev | CYP2C19_108 | gcacacacacttaattagcatgg | tggttaaggatttgctgacatcc | 250 |
| 112 For/Rev | CYP2A6_112 | cagagagggggaggagggt | caacttcctcctccctacca | 237 |
| 107 For/Rev | CYP2C8_107 | cgcagagtagagtcacccac | tgcagcactttcttccttgg | 228 |
| 105 For/Rev | CYP2C9_105 | ctttcttgcctgggatctcc | agtgatatggagtagggtcacc | 223 |
| 111 For/Rev | CYP2B6_111 | caccaccatcctccagaact | gggagtcagagccattgtct | 205 |
| 113 For/Rev | CYP2A6_113 | cttgcccaccccgaagtc | tctctctcccaccctactcc | 198 |
| 101 For/Rev | SLCO1B1_101 | ggaatctgggtcatacatgtgg | agtagacaaagggaaagtgatca | 187 |
| 106 For/Rev | CYP2C8_106 | caagtcttccctacaaccttga | ctccctgcaatgtgatctgc | 185 |
| 102 For/Rev | SLCO1B1_102 | ccagaaataatccagtgacatctca | ttgccacttgaagatttgcaa | 157 |
| 109 For/Rev | CYP2C19_109 | atccggcgtttctccctc | ccagaaaggtcagtgatagagag | 151 |
| 100839\|100840 | GSTM1_Abn | GTGGCATGAAACCAGTACTCAA | GACAGATTCAGGGACAGCATCT | 662 |
| 1-0223\|1-0224 | 2B6_2 | GCTTTACTGGCCCAACCAGA | GGAGGTGTGGGTTGGTTGTAGA | 518 |
| 100653\|100654 | 2B6star4 | TCTCTCCCTGTGACCTGCTA | CTCCCTCTGTCTTTCATTCTGTC | 495 |
| 1-0207\|1-0208 | 2A6_ex5 | GCCCCACTGAAATACCTAAACAAC | GGGCCTGTGTCATCTGCCT | 402 |
| 100833\|100834 | 2A6_17 | CATAGGCGGAGCCATATCAT | GATAGCACCTTAGGGAGGAAGAA | 298 |
| 104 For/Rev | CYP3A7_104 | gctgttggattgtttatatgctgg | cttgagtttgtgataagaacccaga | 237 |
| 110 For/Rev | CYP2B6_110 | ccaggtgtgatctttgccaa | gtctttctattcccgtgcacc | 229 |

6 plex
15 plex
21 plex
28 plex

FIG. 14

**Probeset 1**

AX-112282580
Intensities: called genotypes

**Probeset 2**

AX-112278610
Intensities: called genotypes

■ Magenta: ref, no spike
⊞ Yellow →blue:
increasing concentration
of spike from 0.5-
5000pM

FIG. 15

EP 3 362 580 B1

| | Amplicon Name | Primer Pair PNs | Primer Sequence One[1,2] | Primer Two[1,2] |
|---|---|---|---|---|
| 1 | GSTM1_Abn | 100839\|100840 | GTGGCATGAAACCAGTACTCAA | GACAG**A**TTCAGGGACAGCATCT |
| 2 | 1A2_C | 1-0203\|1-0204 | GCTACACATGATCGAGCTATAC | CAGGTCTCTTCACTGTAAAGTTA |
| 3 | 2D6_5 | 1-0217\|1-0218 | **CC**TCC**A**CC**GG**CCTACC**CT** | CT**GG**TCCAGCCTGTGGTTTC |
| 4 | 2B6star4 | 100653\|100654 | TCTCTCCCTGTGA**CC**TGCTA | C**TC**CCTCTGTCTTTCATTCTGTC |
| 5 | 2D6_1 | 1-0209\|1-0210 | GGC**TAAT**GCCTTCATGG**CC**A | **T**GCA**G**AGACTC**C**TCGGTCTCT |
| 6 | 2D6_3 | 1-0213\|1-0214 | TACCTCCTATCCACGTCAGAGA | CTCATTCCTCCTGGGA**CGC** |
| 7 | 2D6_2 | 1-0211\|1-0212 | CCAAGGTTCAAATAGGACTAGGAC | G**C**TCACACCTCCCTAGTGC |
| 8 | 2A6star9 | 100641\|100642 | CCCTCCTGAAGTACCACAGATT | GGGAGTTGTACATCTGCTCTG**T** |
| 9 | 2A6_ex5 | 1-0207\|1-0208 | **G**CCCCACTGAAATACCTAAACAAC | GGGCCTGTGTCATCTGCCT |
| 10 | 2D6_7 | 100639\|100640 | CAAGAAGGAGTGTCAGGGCC | TGTGGTGGCATTGAGGACT**A** |
| 11 | 2B6ex4 | 100651\|100652 | AG**G**TGACAGCCTGATG**TT**CC | C**A**GGTCTCCATGTCCCTGAT |
| 12 | SULT1A1_ex6 | 100841\|100842 | GT**AA**TC**CG**AGCCTCCAC**T**GA | AG**C**AAAGCTGGAGTCTCAT**CC** |
| 13 | 2D6_4 | 1-0215\|1-0216 | GTCCCGAGTATGCTCT**CGG** | GGGTGTCCCAGCAAAGTTCAT |
| 14 | CYP2C19_108 | 108 For\|Rev | gcacaca**c**acttaa**t**tagcatgg | tg**g**ttaaggatttgctgacatcc |
| 15 | CYP2C8_106 | 106 For\|Rev | caagtcttcc**c**tacaaccttga | ctccctg**ca**atgtgat**c**t**g**c |

1-UCSC databasebuild 142 filtering "All SNPS" identified in blue, "Common SNPs" (>1%) shown in **red**
2-DMET Plus primers carried over into Axiom DMET Plus shown in upper case, newly designed primers in lower case lettering

FIG. 16

EP 3 362 580 B1

| Frequency of Common SNPs | rsID of Common SNPs | Amplicon GRCh37/hg19 Coordinates | Amplicon Size |
|---|---|---|---|
| NA | NA | chr1:110232649-110233310 | 662 |
| NA | NA | chr15:75038086+75038682 | 597 |
| 0.0162 | rs29001678 | chr22:42526521-42527069 | 549 |
| NA | NA | chr19:41515062+41515556 | 495 |
| NA | NA | chr22:42524755-42525233 | 479 |
| 0.0184 | rs76015180 | chr22:42524114-42524561 | 448 |
| NA | NA | chr22:42525581-42525989 | 409 |
| NA | NA | chr19:41356160-41356565 | 406 |
| NA | NA | chr19:41352693-41353094 | 402 |
| NA | NA | chr22:42523328-42523702 | 375 |
| NA | NA | chr19:41512746+41513120 | 375 |
| 0.2909 | rs4149393 | chr16:28617274-28617596 | 323 |
| NA | NA | chr22:42523764-42524014 | 251 |
| NA | NA | chr10:96522379+96522628 | 250 |
| NA | NA | chr10:96824497+96824681 | 185 |

FIG. 16 Cont.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012221255 A1
- US 20140274749 A
- US 536841
- WO 0058516 A
- US 5143854 A
- US 5242974 A
- US 5252743 A
- US 5324633 A
- US 5384261 A
- US 5405783 A
- US 5424186 A
- US 5451683 A
- US 5482867 A
- US 5491074 A
- US 5527681 A
- US 5550215 A
- US 5571639 A
- US 5578832 A
- US 5593839 A
- US 5599695 A
- US 5624711 A
- US 5631734 A
- US 5795716 A
- US 5831070 A
- US 5837832 A
- US 5856101 A
- US 5858659 A
- US 5936324 A
- US 5968740 A
- US 5974164 A
- US 5981185 A
- US 5981956 A
- US 6025601 A
- US 6033860 A
- US 6040193 A
- US 6090555 A
- US 6136269 A
- US 6269846 B
- US 6428752 B
- US 9900730 W
- WO 9936760 A
- US 0104285 W
- US 5412087 A
- US 6147205 A
- US 6262216 B
- US 6310189 B
- US 5889165 A
- US 5959098 A
- US 5800992 A

- US 6013449 A
- US 6020135 A
- US 6040138 A
- US 6177248 B
- US 6309822 B
- US 319253
- US 10013598 B
- US 5856092 A
- US 6300063 B
- US 6284460 B
- US 6361947 B
- US 6368799 B
- US 6333179 B
- US 5871928 A
- US 5902723 A
- US 6045996 A
- US 5541061 A
- US 6197506 B
- US 4683202 A
- US 4683195 A
- US 4800159 A
- US 4965188 A
- US 5333675 A
- US 6300070 B
- WO 8810315 A
- WO 9006995 A
- US 6410276 B
- US 4437975 A
- US 5413909 A
- US 5861245 A
- US 5409818 A
- US 5554517 A
- US 6063603 A
- US 8700880 W
- US 5648245 A
- US 5242794 A
- US 5494810 A
- US 4988617 A
- US 20030143599 A
- US 6391592 B
- US 5874219 A
- US 6386749 B
- US 6391623 B
- US 5834758 A
- US 6141096 A
- US 6185030 B
- US 6201639 B
- US 6218803 B
- US 6225625 B

- US 364731
- US 9906097 W
- WO 9947964 A
- US 5547839 A
- US 5733729 A
- US 6066454 A
- US 6185561 B
- US 6188783 B
- US 6223127 B
- US 6229911 B
- US 6308170 B
- US 20050250151 A
- US 20050244883 A
- US 20050108197 A
- US 20050079536 A
- US 20050042654 A
- US 063559
- US 60349546 B
- US 60376003 B
- US 60394574 B

- US 60403381 B
- US 6156501 A
- US 5445934 A
- US 5744305 A
- US 5677195 A
- US 5770358 A
- US 5789162 A
- US 5708153 A
- US 20080131894 A
- US 6582908 B
- EP 235726 A, Dattagupta
- WO 8911548 A
- US 6858412 B
- US 8716190 B
- US 8828688 B
- US 8759036 B
- US 20130296172 A
- US 20150284786 A
- US 6558928 B
- US 7074564 B

**Non-patent literature cited in the description**

- Using Antibodies: A Laboratory Manual, Cells: A Laboratory Manual, PCR Primer: A Laboratory Manual, and Molecular Cloning: A Laboratory Manual. Genome Analysis: A Laboratory Manual Series. Cold Spring Harbor Laboratory Press
- **STRYER, L.** Biochemistry. Freeman, 1995
- **GAIT.** Oligonucleotide Synthesis: A Practical Approach. IRL Press, 1984
- **COX.** Lehninger, Principles of Biochemistry. W.H. Freeman Pub, 2000
- **BERG et al.** Biochemistry. W.H. Freeman Pub, 2002
- PCR Technology: Principles and Applications for DNA Amplification. Freeman Press, NY, 1992
- PCR Protocols: A Guide to Methods and Applications. Academic Press, 1990
- **MATTILA et al.** *Nucleic Acids Res.,* 1991, vol. 19, 4967
- **ECKERT et al.** *PCR Methods and Applications,* 1991, vol. 1, 17
- PCR. IRL Press
- **WU ; WALLACE.** *Genomics,* 1989, vol. 4, 560
- **LANDEGREN et al.** *Science,* 1988, vol. 241, 1077
- **BARRINGER et al.** *Gene,* 1990, vol. 89, 117
- **KWOH et al.** *Proc. Natl. Acad. Sci. USA,* 1989, vol. 86, 1173
- **GUATELLI et al.** *Proc. Nat. Acad. Sci. USA,* 1990, vol. 87, 1874
- **WALKER et al.** *1992, Nucleic Acids Res.,* 1992, vol. 20 (7), 1691-6
- **MAKRIGIORGOS et al.** *Nat Biotechnol,* 2002, vol. 20, 936-9

- **DONG et al.** *Genome Research,* 2001, vol. 11, 1418
- **MANIATIS et al.** Molecular Cloning: A Laboratory Manual. Cold Spring Harbor, 1989
- Guide to Molecular Cloning Techniques. **BERGER ; KIMMEL.** Methods in Enzymology. Academic Press, Inc, 1987, vol. 152
- **YOUNG ; DAVISM.** *P.N.A.S,* 1983, vol. 80, 1194
- **SETUBAL ; MEIDANIS et al.** Introduction to Computational Biology Methods. PWS Publishing Company, 1997
- Computational Methods in Molecular Biology. Elsevier, 1998
- **RASHIDI ; BUEHLER.** Bioinformatics Basics: Application in Biological Science and Medicine. CRC Press, 2000
- Ouelette and Bzevanis Bioinformatics: A Practical Guide for Analysis of Gene and Proteins. Wiley & Sons, 2001
- **ALBERT L.** Lehninger, Principles of Biochemistr. Worth Pub, 1982, 793-800
- *Gene Expression Technical Manual,* 2004
- **SAMBROOK et al.** Molecular Cloning: A Laboratory Manual. Cold Spring Harbor Laboratory Press, 2001
- **FODOR et al.** *Science,* 1991, vol. 251, 767-777
- **SAIKI et al.** *Nature,* 1986, vol. 324, 163-166
- **HOFFMANN et al.** Next generation genome-wide association tool: design and coverage of a high-throughput European-optimized SNP array. *Genomics,* 2011, vol. 98 (2), 79-89

- **HOFFMANN et al.** Design and coverage of high throughput genotyping arrays optimized for individuals of East Asian, African American, and Latino race/ethnicity using imputation and a novel hybrid SNP selection algorithm. *Genomics,* 2011, vol. 98 (6), 422-30
- **GUNDERSON et al.** Whole-genome genotyping of haplotype tag single nucleotide polymorphisms. *Pharmacogenomics,* 2006, vol. 7 (4), 641-8
- **STEEMERS et al.** Whole-genome genotyping with the single-base extension assay. *Nature Methods,* 2006, vol. 3, 31-33
- **HARDENBOL et al.** *Nat. Biotechnol,* 2003, vol. 21, 673-8
- **HARDENBOL et al.** *Genome Res.,* 2005, vol. 15, 269-275
- **JI et al.** *Cancer Res.,* vol. 66, 7910-9